# EUROPEAN PATENT SPECIFICATION

(11) **EP 1 080 208 B1**
(45) Date of publication and mention of the grant of the patent: **30.03.2005**
(21) Application number: 99926414.6
(22) Date of filing: 25.05.1999
(51) Int. Cl.: C12N 15/63, C12N 15/82, A01H 5/00

(54) **DSRNA-MEDIATED REGULATION OF GENE EXPRESSION IN PLANTS**
DSRNA-BEDINGTE REGULATION DER GEN-EXPRESSION IN PFLANZEN
REGULATION ASSUREE PAR L'ARN A DOUBLES BRINS DE L'EXPRESSION GENETIQUE DANS LES PLANTES

(30) Priority: 26.05.1998 US 84942
(43) Date of publication of application: 07.03.2001
(60) Divisional application: 04030122.8
(73) Proprietor: Syngenta Participations AG, 4058 Basel (CH)
(72) Inventor: HEIFETZ, Peter, Bernard, San Diego, CA 92131 (US); PATTON, David, Andrew, Durham, NC 27703 (US); LEVIN, Joshua, Zvi, Durham, NC 27701 (US); QUE, Qiudeng, Apex, NC 27502 (US); DE FRAMOND, Annick, Jeanne, Pittsboro, NC 27312 (US); DUNN, Martha, M., Hillsborough, NC 27278 (US); CHEN, Jeng, Shong, Chapel Hill, NC 27514 (US)
(74) Representative: Bastian, Werner Maria
(86) International application number: PCT/EP1999/003608
(87) International publication number: WO 1999/061631

(56) References cited:
- EP-A- 0 458 367
- WO-A-94/17176
- WO-A-99/15682
- WO-A-99/32619
- FIRE A ET AL: "Potent and specific genetic interference by double-stranded RNA in Caenorhabditis elegans" NATURE, vol. 391, 19 February 1998 (1998-02-19), pages 806-811, XP002095876 ISSN: 0028-0836
- DALE ET AL: "Intra- and intermolecular site-specific recombination in plant cells mediated by bacteriophage P1 recombinase" GENE, vol. 91, 1990, pages 79-85, XP002094597 ISSN: 0378-1119
- WASSENEGGER, M. & P LISSIER, T.: "A model for RNA-mediated gene silencing in higher plants" PLANT MOLECULAR BIOLOGY., vol. 37, May 1998 (1998-05), pages 349-362, XP002114471 ISSN: 0167-4412
- MATZKE M A ET AL: "HOW AND WHY DO PLANTS INACTIVATE HOMOLOGOUS (TRANS)GENES?" PLANT PHYSIOLOGY, vol. 107, no. 3, 1 March 1995 (1995-03-01), pages 679-685, XP002021174 ISSN: 0032-0889
- STAM ET AL: "Post-transcriptional silencing of chalcone synthase in Petunia by inverted transgene repeats" PLANT JOURNAL, vol. 12, July 1997 (1997-07), pages 63-82, XP002075455 ISSN: 0960-7412
- WATERHOUSE, P. ET AL.: "Virus resistance and gene silencing in plants can be induced by simultaneous expression of sense and antisense RNA" PROCEEDINGS OF THE NATIONAL ACADEMY OF SCIENCES OF USA., vol. 95, November 1998 (1998-11), pages 13959-13964, XP002114472 ISSN: 0027-8424
- MONTGOMERY M K ET AL: "Double-stranded RNA as a mediator in sequence-specific genetic silencing and co-suppression" TRENDS IN GENETICS, vol. 14, no. 7, 1 July 1998 (1998-07-01), page 255-258 XP004124680 ISSN: 0168-9525

### SEQUENCE LISTING

<110> Novartis AG
<120> Regulation of Gene Expression
<130> S-30496A
<140>
   <141>
<150> US 09/084,942
   <151> 1998-05-26
<160> 18
<170> PatentIn Ver. 2.0
<210> 1
   <211> 27
   <212> DNA
   <213> Artificial Sequence
<220>
   <223> Description of Artificial Sequence:
   oligonucleotide
<400> 1
   cgcggatcct ggaagacgcc aaaaaca 27
<210> 2
   <211> 38
   <212> DNA
   <213> Artificial Sequence
<220>
   <223> Description of Artificial Sequence:
   oligonucleotide
<400> 2
   cggaagctta ggctcgccta atcgcagtat ccggaatg 38
<210> 3
   <211> 28
   <212> DNA
   <213> Artificial Sequence
<220>
   <223> Description of Artificial Sequence: oligonucleotide
<400> 3
   cggtctagag gaagacgcca aaaacata 28
<210> 4
   <211> 38
   <212> DNA
   <213> Artificial Sequence
<220>
   <223> Description of Artificial Sequence:
   oligonucleotide
<400> 4
   cggaagctta ggctcgccta atcgcagtat ccggaatg 38
<210> 5
   <211> 22
   <212> DNA
   <213> Artificial Sequence
<220>
   <223> Description of Artificial Sequence:
   oligonucleotide
<400> 5
   gtacctcgag tctagactcg ag 22
<210> 6
   <211> 27
   <212> ENA
   <213> Artificial Sequence
<220>
   <223> Description of Artificial Sequence:
   oligonucleotide
<400> 6
   gatcgagctc cacgagaact gtctccg 27
<210> 7
   <211> 27
   <212> DNA
   <213> Artificial Sequence
<220>
   <223> Description of Artificial Sequence:
   oligonucleotide
<400> 7
   tcagccatgg gaagacaagt acattgc 27
<210> 8
   <211> 27
   <212> DNA
   <213> Artificial Sequence
<220>
   <223> Description of Artificial Sequence:
   oligonucleotide
<400> 8
   cttgccatgg cacgagaact gtctccg 27
<210> 9
   <211> 28
   <212> DNA
   <213> Artificial Sequence
<220>
   <223> Description of Artificial Sequence:
   oligonucleotide
<400> 9
   catggagctc gaagacaagt acattgca 28
<210> 10
   <211> 43
   <212> DNA
   <213> Artificial Sequence
<220>
   <223> Description of Artificial Sequence: oligonucleotide
<400> 10
   catcgagctc ctctgtttaa accacgagaa ctgtctccgt cgc 43
<210> 11
   <211> 20
   <212> DNA
   <213> Artificial Sequence
<220>
   <223> Description of Artificial Sequence:
   oligonucleotide
<400> 11
   tttggagagg acagacctgc 20
<210> 12
   <211> 24
   <212> DNA
   <213> Artificial Sequence
<220>
   <223> Description of Artificial Sequence:
   oligonucleotide
<400> 12
   ggattttggt tttaggaatt agaa 24
<210> 13
   <211> 30
   <212> DNA
   <213> Artificial Sequence
<220>
   <223> Description of Artificial Sequence:
   oligonucleotide
<400> 13
   cgcggatcca agattcaaag tgcgctgctg 30
<210> 14
   <211> 30
   <212> DNA
   <213> Artificial Sequence
<220>
   <223> Description of Artificial Sequence:
   oligonucleotide
<400> 14
   gcgaagcttg gcgacgtaat ccacgatctc 30
<210> 15
   <211> 30
   <212> DNA
   <213> Artificial Sequence
<220>
   <223> Description of Artificial Sequence:
   oligonucleotide
<400> 15
   cggtctagaa agattcaaag tgcgctgctg 30
<210> 16
   <211> 30
   <212> DNA
   <213> Artificial Sequence
<220>
   <223> Description of Artificial Sequence:
   oligonucleotide
<400> 16
   gcgaagcttg gcgacgtaat ccacgatctc 30
<210> 17
   <211> 29
   <212> DNA
   <213> Artificial Sequence
<220>
   <223> Description of Artificial Sequence:
   oligonucleotide
<400> 17
   gcgaagcttg atccatgagc ccagaacga 29
<210> 18
   <211> 27
   <212> DNA
   <213> Artificial Sequence
<220>
   <223> Description of Artificial Sequence:
   oligonucleotide
<400> 18
   gccaagcttc ctagaacgcg tgatctc 27

## Description

The present invention relates to methods of altering the expression of genes in plants, in particular using sense and antisense RNA fragments of said genes, and to plants with altered gene expression obtained using the methods of the present invention.

Developments in the techniques of molecular biology and plant transformation have allowed the production of transgenic plants with various desirable traits, such as, e.g., resistance to insects and fungal or microbial pathogens, tolerance to herbicides or value-added traits. These desirable traits are mainly obtained by overexpression of a transgene in the plant. However, in some cases, it is also desirable to modify plants so that the expression of a particular gene is altered to create plants with desirable phenotypes or properties of commercial interest. Current methods to alter the expression of a gene usually rely upon techniques of sense or antisense suppression. For example, sense suppression of a chalcone synthase gene in Petunia results in flowers with altered pigmentation and antisense suppression of a polygalacturonidase gene in tomato leads to delayed fruit ripening. Unfortunately, these methods are often variable and unpredictable in their ability to alter gene expression, and in many cases a complete disruption of the particular gene activity is not achieved. Other methods to after gene expression include the use of catalytic ribonucleotides or ribozymes, which can be technically challenging, or homologous gene disruption, which, although the most desirable genetically, is unfortunately not efficient enough with currently available techniques to be routinely used for such purposes.

There is therefore a long-felt but unfulfilled need for novel methods allowing one to effectively and predictably alter the expression of a gene in plant cells to obtain plants with improved and commercially important properties.

The present invention relates to the production of plants with improved properties and traits using molecular techniques and genetic transformation. In particular, the invention relates to methods of altering the expression of a gene in a plant cell using sense and antisense RNA fragments of the gene. Importantly, such sense and antisense RNA fragments are capable of forming a double-stranded RNA molecule. The invention also relates to plant cells obtained using such methods, to plants derived from such cells, to the progeny of such plants and to seeds derived from such plants. In such plant cells or plants, the alteration of the gene expression of a particular gene is more effective, selective and more predicatable than the alteration of the gene expression of a particular gene obtained using current methods known in the art.

EP 0 458 367 is concerned with a technique of silencing the expression of a gene by introducing antisense copies of a target gene into a cell.

WO 99/32619 is concerned with the use of double-stranded RNA to manipulate gene expression in the nematode *C*. *elegans.*

*Fire et al.* (Nature 1998. vol. 391, pp 806-811) is also concened with the use of double-stranded RNA to manipulate gene expression in *C. elegans.*

WO 99/15682 is concerned with the gene silencing in plants using a method termed 'transiently induced gene silencing' (TIGS), which utilises a foreign initiator nucleic acid or nucleic acid complex that is transiently introduced into a cell.

The invention therefore provides:
A method for altering expression of a target gene in a plant cell comprising introducing into a plant cell a sense RNA fragment of a target gene and an antisense RNA fragment of said target gene, wherein said sense RNA fragment and said antisense RNA fragment are capable of forming a double-stranded RNA molecule. In a preferred embodiment, the RNA fragments are comprised in two different RNA molecules. In another preferred embodiment, the RNA fragments are mixed before being introduced into said cell. In another preferred embodiment, the RNA fragments are mixed before being introduced in said cell under conditions allowing them to form a double-stranded RNA molecule. In another preferred embodiment, the RNA fragments are introduced into said cell sequentially. In yet another preferred embodiment, the RNA fragments are comprised in one RNA molecule. In such case, the RNA molecule is preferably capable of folding such that said RNA fragments comprised therein form a double-stranded RNA molecule.

The invention further provides:
A method for altering expression of a target gene in a plant cell comprising introducing into a plant cell a first DNA sequence capable of expressing in said cell a sense RNA fragment of a target gene, and a second DNA sequence capable of expressing in said cell an antisense RNA fragment of said target gene, wherein said first DNA sequence and said second DNA sequence are comprised in two different DNA molecules and said first DNA sequence and said second DNA sequence are stably integrated in the genome of said plant cell and said sense RNA fragment and said antisense RNA fragment are capable of forming a double-stranded RNA molecule. In a preferred embodiment, the DNA molecule further comprises a promoter operably linked to said first or said second DNA sequence.

In yet another preferred embodiment, the DNA molecule further comprises a first promoter operably linked to said first DNA sequence and a second promoter operably linked to said second DNA sequence, wherein the first promoter and the second promoter comprise the same promoter or comprise different promoters.

In another preferred embodiment, the target gene is a native gene of said plant cell, preferably an essential gene of said plant cell. In yet another preferred embodiment, a promoter in the DNA molecule comprises the native promoter of said native target gene. In a further preferred embodiment, the promoter is a heterologous promoter, for example a tissue specific promoter, a developmentally regulated promoter, a constitutive promoter or an inducible promoter. Optionally, the promoter is a divergent promoter capable of initiating transcription of DNA sequences on each side of the promoter. In another preferred embodiment, the gene is a heterologous gene in said plant cell, preferably stably integrated in the genome of said plant cell or, alternatively, present in said plant cell as an extrachromosomal molecule.

The invention also further provides:
A plant cell comprising the sense and antisense RNA fragments of the present invention, wherein the expression of said target gene in said plant cell is altered by said RNA fragments, a plant and the progeny thereof derived from the plant cell, and seeds derived from the plant.

The invention also further provides:
A plant cell obtained by a method of the present invention.

A "double-stranded RNA (dsRNA)" molecule comprises a sense RNA fragment of a target gene and an antisense RNA fragment of the same target gene, which both comprise nucleotide sequences complementary to one another, thereby allowing the sense and antisense RNA fragments to pair and form a double-stranded RNA molecule.

"Complementary" refers to two nucleotide sequences which comprise antiparallel nucleotide sequences capable of pairing with one another upon formation of hydrogen bonds between the complementary base residues in the antiparallel nucleotide sequences.

"Antiparallel" refers herein to two nucleotide sequences paired through hydrogen bonds between complementary base residues with phosphodiester bonds running in the 5'-3' direction in one nucleotide sequence and in the 3'-5' direction in the other nucleotide sequence.

A "target gene" is any gene in a plant cell. For example, a target gene is a gene of known function or is a gene whose function is unknown, but whose total or partial nucleotide sequence is known. Alternatively, the function of a target gene and its nucleotide sequence are both unknown. A target gene is a native gene of the plant cell or is a heterologous gene which had previously been introduced into the plant cell or a parent cell of said plant cell, for example by genetic transformation. A heterologous target gene is stably integrated in the genome of the plant cell or is present in the plant cell as an extrachromosomal molecule, e.g. as an autonomously replicating extrachromosomal molecule.

A "native" gene refers to a gene which is present in the genome of the untransformed plant cell.

An "essential" gene is a gene encoding a protein such as e.g. a biosynthetic enzyme, receptor, signal transduction protein, structural gene product, or transport protein that is essential to the growth or survival of the plant.

To "alter" the expression of a target gene in a plant cell means that the level of expression of the target gene in a plant cell after applying a method of the present invention is different from its expression in the cell before applying the method. To alter gene expression preferably means that the expression of the target gene in the plant is reduced, preferably strongly reduced, more preferably the expression of the gene is not detectable. The alteration of the expression of an essential gene may result in a knockout mutant phenotype in plant cells or plants derived therefrom.

"Isolated" is, in the context of the present invention, an isolated nucleic acid molecule that, by the hand of man, exists apart from its native environment and is therefore not a product of nature. An isolated nucleic acid molecule may exist in a purified form or may exist in a non-native environment such as, for example, a transgenic host cell.

"Expression cassette" as used herein means a DNA sequence capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. Typically, however, the expression cassette is heterologous with respect to the host, i.e., the particular DNA sequence of the expression cassette does not occur naturally in the host cell and must have been introduced into the host cell or an ancestor of the host cell by a transformation event. The expression of the nucleotide sequence in the expression cassette may be under the control of a constitutive promoter or of an inducible promoter which initiates transcription only when the host cell is exposed to some particular external stimulus. In the case of a multicellular organism, such as a plant, the promoter can also be specific to a particular tissue or organ or stage of development.
"Heterologous" as used herein means "of different natural origin" or represents a non-natural state. For example, if a host cell is transformed with a nucleic acid sequence derived from another organism, particularly from another species, that nucleic acid sequence is heterologous with respect to that host cell and also with respect to descendants of the host cell which carry that nucleic acid sequence. Similarly, heterologous refers to a nucleotide sequence derived from and inserted into the same natural, original cell type, but which is present in a non-natural state, e.g. a different copy number, or under the control of different regulatory elements.

In its broadest sense, the term "substantially similar", when used herein with respect to a nucleotide sequence, means a nucleotide sequence corresponding to a reference nucleotide sequence, wherein the corresponding sequence encodes a polypeptide having substantially the same structure and function as the polypeptide encoded by the reference nucleotide sequence, e.g. where only changes in amino acids not affecting the polypeptide function occur. Desirably the substantially similar nucleotide sequence encodes the polypeptide encoded by the reference nucleotide sequence. The percentage of identity between the substantially similar nucleotide sequence and the reference nucleotide sequence (number of complementary bases in the complementary sequence divided by total number of bases in the complementary sequence) desirably is at least 80%, more desirably 85%, preferably at least 90%, more preferably at least 95%, still more preferably at least 99%.
"Regulatory elements" refer to sequences involved in conferring the expression of a nucleotide sequence. Regulatory elements comprise a promoter operably linked to the nucleotide sequence of interest and termination signals. They also typically encompass sequences required for proper translation of the nucleotide sequence.

A "plant" refers to any plant or part of a plant at any stage of development. Therein are also included cuttings, cell or tissue cultures and seeds. As used in conjunction with the present invention, the term "plant tissue" includes, but is not limited to, whole plants, plant cells, plant organs, plant seeds, protoplasts, callus, cell cultures, and any groups of plant cells organized into structural and/or functional units.

The present invention relates to methods for regulating gene expression in plant cells. Commonly available methods to regulate the expression of a gene in plant cells lack predictability and show variability depending upon which gene is to be regulated. The present method alleviates these problems and provides for reproducible and efficacious regulation of a gene in plant cells.
The present invention utilizes a sense RNA fragment and an antisense RNA fragment of a target gene to alter the expression of the gene in a plant cell. In a first embodiment, the invention provides a method for altering expression of a target gene in a plant cell comprising introducing into a plant cell a sense RNA fragment of a target gene and an antisense RNA fragment of said target gene, wherein said sense RNA fragment and said antisense RNA fragment are capable of forming a double-stranded RNA molecule, wherein the expression of said target gene in said cell is altered. In a preferred embodiment, the RNA fragments are introduced in the plants cell by different transformation methods. For example, the RNA fragments are transferred to the host cells using particle bombardment as described in co-pending application 08/717,676. In another preferred embodiment, the RNA fragments are introduced into the protoplasts or other types of cells by PEG-mediated transformation as described in Lebel et al. (1995) Theor. Appl. Genet 91: 899-906 or by electroporation. In another preferred embodiment, other techniques, such as microinjection of the RNA fragments, are used.
In another preferred embodiment, the RNA fragments are comprised in two different RNA molecules. In this case, the RNA fragments are mixed before being introduced into said cell, e.g. under conditions allowing them to form a double-stranded RNA molecule. In another preferred embodiment, the RNA fragments are introduced into said cell sequentially. Preferably, the time interval between the introduction of each of the RNA molecules is short, preferably less than one hour. In yet another embodiment, the RNA fragments are comprised in one RNA molecule. By using one single RNA molecule, the two complementary RNA fragments are in close proximity such that pairing and double strand formation is favored. In such case, the RNA molecule is preferably capable of folding such that said RNA fragments comprised therein form a double-stranded region. In this case, the complementary parts of the RNA fragments recognize one another, pair with each other and form the double-stranded RNA molecule. In a preferred embodiment, the RNA fragments are incubated under conditions allowing them to form a double-stranded RNA molecule prior to introduction into the cell. In yet another embodiment, the RNA molecule comprises a linker between the sense RNA fragment and the antisense RNA fragment. The linker preferably comprises a RNA sequence encoded by an expression cassette comprising a functional gene, e.g. a selectable marker gene. In another embodiment, the linker comprises a RNA sequence encoded by regulatory sequences, which e.g. comprise intron processing signals.
In a further preferred embodiment, the present invention provides a method for altering expression of a target gene in a plant cell comprising introducing into a plant cell a first DNA sequence capable of expressing in said cell a sense RNA fragment of a target gene and a second DNA sequence capable of expressing in said cell an antisense RNA fragment of said target gene, wherein said first DNA sequence and said second DNA sequence are comprised in two different DNA molecules and said first DNA sequence and said second DNA sequence are stably integrated in the genome of said plant cell and said sense RNA fragment and said antisense RNA fragment are capable of forming a double-stranded RNA molecule.
In the present invention, the DNA sequences are comprised in two different DNA molecules.
The first DNA sequence is operably linked to a first promoter and the second DNA sequence is operably linked to a second promoter. In a preferred embodiment, the two promoters comprise the same promoter or, alternatively, comprise different promoters. Termination signal are also optionally included in the DNA molecules.

In the DNA molecules of the present invention, the DNA sequences are preferably operably linked to promoters. In a preferred embodiment, a promoter in the DNA molecule comprises a native promoter of said native target gene to be inactivated, in order to insure that the double-stranded RNA is present in the same tissues and at the same time in development as is the target gene is expressed. In another embodiment the promoter is a heterologous promoter, for example a tissue specific promoter, a developmentally regulated promoter, a constitutive promoter, divergent or an inducible promoter. In another preferred embodiment the gene is an heterologous gene in said plant cell. Termination signal are also optionally included in the DNA molecules.

The two distinct RNA molecules are preferably capable of forming a double-stranded region, in which the complementary parts of the RNA fragments recognize one another, pair with each other and form the double-stranded RNA molecule. DNA molecules of the present invention are transformed into plant cells using methods well-known in the art or described below. For example, microprojectile bombardment, microinjection or Agrobacterium-mediated transformation is used. Also, transformation of protoplasts with DNA molecules of the present invention using electroporation or chemical treatment (e.g. PEG treatment) is used.
Alternatively, viral vectors are used to introduce DNA molecules or RNA fragments of the present invention into plant cells, e.g. through so-called agroinfection. In another preferred embodiment, DNA molecules or RNA fragments of the present invention into plant cells are introduced into plant cells by vacuum infiltration or by rubbing on leat surface.
The methods can result in plant cells comprising the sense and antisense RNA fragments of the present invention, wherein the expression of said target gene in said plant cell is altered by said RNA fragments, a plant and the progeny thereof derived from the plant cell, and seeds derived from the plant.
In the present invention the length of the complementary region between the sense and antisense RNA fragments comprises desirably at least 15 nucleotides long, more desirably at least 50 nucleotides long, preferably at least 500 bp long. Preferably, the complementary region is less than 5 kb, more preferably less than 2 kb. Optionally, the complementary region between the sense and antisense RNA fragments comprises the coding region of the target gene. In other preferred embodiment, the complementary region comprises untranslated regions (UTR) of the target gene, e.g. 5' UTR or 3' UTR. In another preferred embodiment, a DNA sequence encoding a sense or antisense RNA fragment of the present invention is derived from a c-DNA molecule. In another embodiment, the complementary sequences comprise regulatory elements of the target gene whose expression is altered, such as promoter or termination signals.
In another preferred embodiment, the complementary region between the sense and antisense RNA fragments is identical to the corresponding sequence of the gene whose expression is altered. In another preferred embodiment, the complementary region between the sense and antisense RNA fragments is substantially similar to the corresponding sequence of the gene whose expression is altered and is still capable of altering the expression of the gene. In this case, the complementary region is desirably at least 50% identical to the corresponding sequence of the gene whose expression is altered more desirably at least 70% identical, preferably at least 90% identical, more preferably at least 95% identical. Thereby, using a single double-stranded RNA molecule allows to alter the expression of a single gene or of a plurality of genes, the single gene comprising sequences identical to the double-stranded RNA or being substantially similar to the double-stranded RNA.
In another preferred embodiment, the complementary region between the sense and antisense RNA fragments does not contain any mismatch between the sense and antisense RNA fragments. In another preferred embodiment, the complementary region between the sense and antisense RNA fragments comprises at least one mismatch between the sense and antisense RNA fragments, and the two RNA fragments are still capable of pairing and forming a double-stranded RNA molecule, thereby altering the expression of the gene. Desirably, there is less than 50% mismatch between the sense and antisense RNA fragments in the complementary region, more desirably less than 30% mismatch, preferably less than 20% mismatch, more preferably less than 10% mismatch, yet more preferably less than 5 % mismatch.

A method of the present invention is used for example to alter the expression of a gene involved in a metabolic pathway of a plant cell, in resistance or susceptibility of a plant to diseases or in cell differentiation. Such alterations result in plants with commercially important improved traits, such as modifications of the particular metabolic pathway, resistance to diseases or changes in cell differentiation. Other examples of target genes are described e.g. in US patent 5,107,065, 5,283,184 and 5,034,323, herein incorporated by reference. A method of the present invention is also used to alter the expression of a gene in order to unravel its function. In this case, for example, DNA sequences capable of expressing sense and antisense RNA fragments of the gene are introduced into a plant cell by a transformation method well known in the art or described below. The DNA sequences are for example stably integrated in the genome of the plant cell. The plant cell is then examined for e.g. phenotypic or metabolic changes. Alternatively, a plant is regenerated from the plant cell and the plant or its progeny is examined for, e.g., phenotypic or metabolic changes. For example, essential genes are discovered using such a method and screening the transformed plants for e.g. embryo lethality, seedling lethality or other relevant phenotypes. Knowledge of the function of the gene is then used to produce crops with improved properties by genetic transformation or to screen for novel chemicals. In particular, essential genes are good candidates as targets for herbicidal compounds. Essential gene sequences, are for example overexpressed in a plant to confer resistance upon the plant to an herbicidal compound which inhibit the function of the naturally occurring enzyme encoded by the gene. Mutated variants of the essential gene are also produced and screened for tolerance to the herbicidal compound or to related compounds. Such mutated variants are produced by various methods known in the art. The enzyme encoded by the essential gene is also used to screen for compounds which inhibit its function, e.g. in a in-vitro high throughput screen. Inhibiting compounds are then tested for herbicidal activity.
A method of the present invention is also used to randomly alter the expression of genes without prior knowledge of their nucleotide sequence. In this case, a library of random RNA fragments capable of pairing and forming double-stranded RNA molecules or a library of random DNA sequences encoding RNA fragments capable of pairing and forming a double-stranded RNA molecule is prepared and introduced into plant cells using methods well-known in the art or described below. The transformed plant cells or plants are screened for a particular property or phenotype. For example, essential genes as described above are discovered using such screen. Another example of a screen is for unhampered growth, or growth less hampered than untransformed cells under various conditions, such as higher salinity or osmotic pressure, higher temperature, presence of toxic or harmful substances, e.g. herbicidal compounds. After such screen, the double-stranded RNA or the DNA molecule encoding the double-stranded RNA is recovered and the sequence of the complementary RNA fragments is determined, thus allowing to isolate the gene whose alteration of expression is responsible for the particular property or phenotype. Such gene is then used e.g. to improve crops by genetic transformation or to screen for novel chemicals.

### Plant Transformation Technology

DNA molecules of the present invention are incorporated in plant or bacterial cells using conventional recombinant DNA technology. Generally, a DNA molecule of the present invention is comprised in a transformation vector. A large number of such vector systems known in the art are used, such as plasmids, bacteriophage viruses and other modified viruses. The components of the expression system are also modified, e.g. to increase expression of the sense and antisense RNA fragments. For example, truncated sequences, nucleotide substitutions or other modifications are employed. Expression systems known in the art are used to transform virtually any crop plant cell under suitable conditions. A transgene comprising a DNA molecule of the present invention is preferably stably transformed and integrated into the genome of the host cells. In another preferred embodiment, the transgene comprising a DNA molecule of the present invention is located on a self-replicating vector. Examples of self-replicating vectors are viruses, in particular gemini viruses. Transformed cells are preferably regenerated into whole plants.
Plants transformed in accordance with the present invention may be monocots or dicots and include, but are not limited to, maize, wheat, barley, rye, sweet potato, bean, pea, chicory, lettuce, cabbage, cauliflower, broccoli, tumip, radish, spinach, asparagus, onion, garlic, pepper, celery, squash, pumpkin, hemp, zucchini, apple, pear, quince, melon, plum, cherry, peach, nectarine, apricot, strawberry, grape, raspberry, blackberry, pineapple, avocado, papaya, mango, banana, soybean, tomato, sorghum, sugarcane, sugarbeet, sunflower, rapeseed, clover, tobacco, carrot, cotton, alfalfa, rice, potato, eggplant, cucumber, Arabidopsis, and woody plants such as coniferous and deciduous trees. Once a desired nucleotide sequence has been transformed into a particular plant species, it may be propagated in that species or moved into other varieties of the same species, particularly including commercial varieties, using traditional breeding techniques.

### A. Requirements for Construction of Plant Expression Cassettes

Gene sequences intended for expression in transgenic plants are first assembled in expression cassettes behind a suitable promoter expressible in plants. The expression cassettes may also comprise any further sequences required or selected for the expression of the transgene. Such sequences include e.g., but are not restricted to, transcription terminators, extraneous sequences to enhance expression such as introns, vital sequences, and sequences intended for the targeting of the gene product to specific organelles and cell compartments. These expression cassettes can then be easily transferred to the plant transformation vectors described *infra.* The following is a description of various components of typical expression cassettes.

### 1. Promoters

The selection of the promoter used in expression cassettes determine the spatial and temporal expression pattern of the transgene in the transgenic plant. Selected promoters express transgenes in specific cell types (such as leaf epidermal cells, mesophyll cells, root cortex cells) or in specific tissues or organs (roots, leaves or flowers, for example) and the selection reflects the desired location of accumulation of the gene product. Alternatively, the selected promoter drives expression of the gene under various inducing conditions. Promoters vary in their strength, i.e., ability to promote transcription. Depending upon the host cell system utilized, any one of a number of suitable promoters known in the art is used. For example, for constitutive expression, the CaMV 35S promoter, the rice actin promoter, or the ubiquitin promoter are used. For example, for regulatable expression, the chemically inducible PR-1 promoter from tobacco or *Arabidopsis* is used *(see, e.g.,* U.S. Patent No. 5,689,044).

A preferred category of promoters is that which is wound inducible. Numerous promoters have been described which are expressed at wound sites. Preferred promoters of this kind include those described by Stanford *et al.* Mol. Gen. Genet. 215: 200-208 (1989), Xu *et al.* Plant Molec. Biol. 22: 573-588 (1993), Logemann *et al.* Plant Cell 1: 151-158 (1989), Rohrmeier & Lehle, Plant Molec. Biol. 22: 783-792 (1993), Firek *et al.* Plant Molec. Biol. 22: 129-142 (1993), and Warner *et al.* Plant J. 3: 191-201 (1993).

Preferred tissue specific expression patterns include green tissue specific, root specific, stem specific, and flower specific. Promoters suitable for expression in green tissue include many which regulate genes involved in photosynthesis, and many of these have been cloned from both monocotyledons and dicotyledons. A preferred promoter is the maize PEPC promoter from the phosphoenol carboxylase gene (Hudspeth & Grula, Plant Molec. Biol. 12: 579-589 (1989)). A preferred promoter for root specific expression is that described by de Framond (FEBS 290: 103-106 (1991); EP 0 452 269 and a further preferred root-specific promoter is that from the T-1 gene provided by this invention. A preferred stem specific promoter is that described in US patent 5,625,136 and which drives expression of the maize *trpA* gene.

Preferred embodiments of the invention are transgenic plants expressing nucleotide sequence in a root-specific fashion. Further preferred embodiments are transgenic plants expressing the nucleotide sequence in a wound-inducible or pathogen infection-inducible manner.

### 2. Transcriptional Terminators

A variety of transcriptional terminators are available for use in expression cassettes. These are responsible for the termination of transcription beyond the transgene and its correct polyadenylation. Appropriate transcriptional terminators are those that are known to function in plants and include the CaMV 35S terminator, the *tml* terminator, the nopaline synthase terminator and the pea *rbcS* E9 terminator. These are used in both monocotyledonous and dicotyledonous plants.

### 3. Sequences for the Enhancement or Regulation of Expression

Numerous sequences have been found to enhance gene expression from within the transcriptional unit and these sequences can be used in conjunction with the genes of this invention to increase their expression in transgenic plants. For example, various intron sequences such as introns of the maize *Adhl* gene have been shown to enhance expression, particularly in monocotyledonous cells. In addition, a number of non-translated leader sequences derived from viruses are also known to enhance expression, and these are particularly effective in dicotyledonous cells.

### 4. Coding Sequence Optimization

The coding sequence of the selected gene may be genetically engineered by altering the coding sequence for optimal expression in the crop species of interest. Methods for modifying coding sequences to achieve optimal expression in a particular crop species are well known (see, *e.g.* Perlak *et al., Proc. Natl. Acad. Sci. USA 88:* 3324 (1991); and Koziel *et al., Bio*/*technol. 11:* 194 (1993)).

In another preferred embodiment, a DNA molecule of the present invention is directly transformed into the plastid genome. Plastid transformation technology is extensively described in U.S. Patent Nos. 5,451,513, 5,545,817, and 5,545,818, in PCT application no. WO 95/16783, and in McBride *et al.* (1994) Proc. Natl. Acad. Sci. USA 91, 7301-7305. The basic technique for chloroplast transformation involves introducing regions of cloned plastid DNA flanking a selectable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate homologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab, Z., Hajdukiewicz, P., and Maliga, P. (1990) Proc. Natl. Acad. Sci. USA 87, 8526-8530; Staub, J. M., and Maliga, P. (1992) Plant Cell 4, 39-45). The presence of cloning sites between these markers allowed creation of a plastid targeting vector for introduction of foreign DNA molecules (Staub, J.M., and Maliga, P. (1993) *EMBO J.* 12, 601-606). Substantial increases in transformation frequency are obtained by replacement of the recessive rRNA or r-protein antibiotic resistance genes with a dominant selectable marker, the bacterial *aadA* gene encoding the spectinomycin-detoxifying enzyme aminoglycoside-3'-adenyltransterase (Svab, Z., and Maliga, P. (1993) *Proc. Natl. Acad. Sci. USA* 90, 913-917). Previously, this marker had been used successfully for high-frequency transformation of the plastid genome of the green alga *Chlamydomonas reinhardtii* (Goldschmidt-Clermont, M. (1991) *Nucl. Acids Res.* 19: 4083-4089). Other selectable markers useful for plastid transformation are known in the art and encompassed within the scope of the invention.
Plastid expression, in which genes are inserted by homologous recombination into the several thousand copies of the circular plastid genome present in each plant cell. In a preferred embodiment, a DNA of the present invention is inserted into a plastid targeting vector and transformed into the plastid genome of a desired plant host. Plants homoplasmic for plastid genomes containing the DNA molecule of the present invention are obtained, and are preferentially capable of high expression of the DNA molecule. Preferably, sense and antisense RNA fragments encoded by the DNA molecule are capable of pairing and of forming a double-stranded RNA molecules in plant plastids to alter the expression of plastid genes. In a preferred embodiment, the sense and antisense fragments do not comprise any mismatch in the complementary region. In another preferred embodiment, the sense and antisense fragments comprise at least one mismatch in the complementary region. In this case, the DNA sequences in the DNA molecule encoding the RNA fragments are not capable of recombining with each other.

### B. Construction of Plant Transformation Vectors

Numerous transformation vectors available for plant transformation are known to those of ordinary skill in the plant transformation arts, and the genes pertinent to this invention can be used in conjunction with any such vectors. The selection of vector depends upon the preferred transformation technique and the target species for transformation. For certain target species, different antibiotic or herbicide selection markers are preferred. Selection markers used routinely in transformation include the *nptII* gene, which confers resistance to kanamycin and related antibiotics (Messing & Vierra. Gene 19: 259-268 (1982); Bevan et al., Nature 304:184-187 (1983)), the *bar* gene, which confers resistance to the herbicide phosphinothricin (White et al., Nucl. Acids Res 18: 1082 (1990), Spencer et al. Theor. Appl. Genet 79: 625-631 (1990)), the *hph* gene, which confers resistance to the antibiotic hygromycin (Blochinger & Diggelmann, Mol Cell Biol 4: 2929-2931), the *manA* gene, which allows for positive selection in the presence of mannose (Miles and Guest (1984) Gene, 32:41-48; U.S. Patent No. 5,767,378), and the *dhfr* gene, which confers resistance to methotrexate (Bourouis et al., EMBO J. 2(7): 1099-1104 (1983)), and the EPSPS gene, which confers resistance to glyphosate (U.S. Patent Nos. 4,940,935 and 5,188,642).

### 1. Vectors Suitable for Agrobacterium Transformation

Many vectors are available for transformation using *Agrobacterium tumefaciens.* These typically carry at least one T-DNA border sequence and include vectors such as pBIN19 (Bevan, Nucl. Acids Res. (1984). Typical vectors suitable for *Agrobacterium* transformation include the binary vectors pCIB200 and pCIB2001, as well as the binary vector pCIB10 and hygromycin selection derivatives thereof. (See, for example, U.S. Patent No. 5,639,949).

### 2. Vectors Suitable for non-Agrobacterium Transformation

Transformation without the use of *Agrobacterium tumefaciens* circumvents the requirement for T-DNA sequences in the chosen transformation vector and consequently vectors lacking these sequences are utilized in addition to vectors such as the ones described above which contain T-DNA sequences. Transformation techniques that do not rely on *Agrobacterium* include transformation via particle bombardment, protoplast uptake (*e.g.* PEG and electroporation) and microinjection. The choice of vector depends largely on the preferred selection for the species being transformed. Typical vectors suitable for *non-Agrobacterium* transformation include pCIB3064, pSOG19, and pSOG35. (*See*, for example, U.S. Patent No. 5,639,949).

### C. Transformation Techniques

Once the DNA sequence of interest is cloned into an expression system, it is transformed into a plant cell. Methods for transformation and regeneration of plants are well known in the art. For example, Ti plasmid vectors have been utilized for the delivery of foreign DNA, as well as direct DNA uptake, liposomes, electroporation, micro-injection, and microprojectiles. In addition, bacteria from the genus *Agrobacterium* can be utilized to transform plant cells.
Transformation techniques for dicotyledons are well known in the art and include *Agrobacterium*-based techniques and techniques that do not require *Agrobacterium.* Non-*Agrobacterium* techniques involve the uptake of exogenous genetic material directly by protoplasts or cells. This is accomplished by PEG or electroporation mediated uptake, particle bombardment-mediated delivery, or microinjection. In each case the transformed cells are regenerated to whole plants using standard techniques known in the art.
Transformation of most monocotyledon species has now also become routine. Preferred techniques include direct gene transfer into protoplasts using PEG or electroporation techniques, particle bombardment into callus tissue, as well as *Agrobacterium*-mediated transformation.
Plants from transformation events are grown, propagated and bred to yield progeny with the desired trait, and seeds are obtained with the desired trait, using processes well known in the art.

The invention will be further described by reference to the following detailed examples. These examples are provided for purposes of illustration only, and are not intended to be limiting unless otherwise specified.

### EXAMPLES

Standard recombinant DNA and molecular cloning techniques used here are well known in the art and are described by Sambrook, *et al.,* Molecular Cloning, eds., Cold Spring Harbor Laboratory Press, Cold Spring Harbor, NY (1989) and by T.J. Silhavy, M.L. Berman, and L.W. Enquist, Experiments with Gene Fusions, Cold Spring Harbor Laboratory, Cold Spring Harbor, NY (1984) and by Ausubel, F.M. *et al.,* Current Protocols in Molecular Biology, pub. by Greene Publishing Assoc. and Wiley-Interscience (1987).

### Example 1: Regulation of the Expression of a Luciferase Gene

### Construction of a chimeric DNA molecule encoding sense and antisense luciferase RNA fragments (sense/antisense construct)

A 738 bp "sense" oriented fragment of the firefly luciferase gene from plasmid pLuc+ (Promega) is amplified from pPH108 plasmid DNA using oligonucleotide primers ds_Luc1 (5'-CGC GGA TCC TGG AAG ACG CCA AAA ACA-3', SEQ ID NO:1; *BamHI* restriction site underlined) and ds_Luc2 (5'-CGG AAG CTT AGG CTC GCC TAA TCG CAG TAT CCG GAA TG-3', SEQ ID NO:2; *HindIII* restriction site underlined). TurboPfu thermostable DNA polymerase (Stratagene) is used in 50 µl reactions according to the manufacturers protocol with five cycles of 95°C / 1 min, 55°C / 1.5 min, 72°C / 2 min followed by twenty five cycles of 95°C / 1 min, 72°C / 3.5 min. In a similar manner a 737 bp "antisense" oriented fragment of the firefly luciferase gene from plasmid pLuc+ is amplified by PCR from pPH108 plasmid DNA using oligonucleotide primers ds_Luc3 (5'-CGG TCT AGA GGA AGA CGC CAA AAA CAT A-3', SEQ ID NO:3; *Xbal* restriction site underlined) and ds_Luc2 (5'-CGG AAG CTT AGG CTC GCC TAA TCG CAG TAT CCG GAA TG-3', SEQ ID NO:4; *HindIII* restriction site underlined). The resulting DNA fragments are purified by electrophoresis through a 1% Tris-acetate gel made from low-melting point agarose (FMC) followed by phenol-chloroform extraction of the excised gel slices containing the PCR products. DNA from the sense product (ds_Luc1/2) is digested with *BamHI* and *HindIII* and DNA from the antisense product (ds_Luc3/2) is digested with *Xbal* and *HindIII* according to standard methods (restriction enzymes were obtained from New England Biolabs). The resulting sticky-ended DNA fragments are gel purified as described above. A DNA fragment containing the *mas 1'* promoter (Velten et al. (1984) EMBO J. 3: 2723-2730) is obtained by digesting plasmid CSA104 with *EcoRI* and *HincII* and purifying a 564 bp DNA fragment. This fragment is redigested with *BamHI* and the 484 bp *EcoRI - BamHI* sub-fragment containing the *mas* 1' promoter isolated and gel purified. In order to construct plasmid pPH169. DNA from cloning vector pLitmus29 (New England Biolabs) is digested with *EcoRI* and *Xbal,* and the isolated fragment is ligated in a four-way reaction using T4 DNA ligase (New England Biolabs) to the *mas 1'* promoter *EcoRI - BamHl* fragment and the sense *(BamHI - HindIII)* and antisense *(HindIII - XbaI)* ds_Luc luciferase gene fragments.

In order to construct binary vector pPH170 for Agrobacterium-mediated plant transformation with the ds_Luc1/2l3 sense/antisense construct, DNA from binary plasmid pSGCHC1 carrying a kanamycin resistance gene for bacterial selection and a hygromycin resistance gene for transgenic plant selection is digested with *EcoRI* and *Xbal.* The resulting 11.6 kb isolated fragment from pSGCHC1 is ligated in a four-way reaction using T4 DNA ligase (New England Biolabs) to the *mas 1*' promoter *EcoRI* - *BamHI* fragment and the sense *(BamHl - HindIII*) and antisense *(Hindlll - XbaI)* ds_Luc luciferase gene fragments.

### Transformation of Agrobacterium and Vacuum-infiltration of Arabidopsis plants

Plasmids pPH170 is introduced into *Agrobacterium tumefaciens* GV3101 by electroporation and transformed colonies selected and amplified. Four to five week old plants of *Arabidopsis thaliana* mutant lines expressing luciferase either constitutively (UBQ3 promoter (Norris et al. (1993) PMB 21: 895-906) / UBQ3 + CaMV 35S 5' UTR/luc+; *pPH108*) or inducibly (Arabidopsis PR-1 promoter/luc+; *pPH135, line 6E*) are vacuum infiltrated with Agrobacterium clones carrying the pPH170 binary T-DNA vector. Transformed plants are co-selected on hygromycin and kanamycin and grown under controlled phytotron conditions for determination of luciferase activity. In addition, luciferase activity in the *pPH135-6E* background is assessed 48 hr after induction with BTH (BTH treatment essentially as described in Lawton et al. Plant J. 10: 71-82). Luciferase activity is quantified using a luminescence-based assay in tissue extracts following the addition of luciferin substrate. Luciferase activity is also monitored *in planta* using a CCD-cooled video imaging system (Hamamatsu).

### Example 2. Regulation of the Expression of the Arabidopsis GL1 Gene

The GL1 gene encodes a myb-like transcription factor that is required for initiation of normal trichome (leaf hair) formation (Oppenheimer et al. (1991) Cell 67: 483-493). Knock out of GL1 expression early in development results in plants lacking trichomes. The knockout phenotype is easy to identify in young seedlings and is not lethal. Three vectors for constitutive expression and three vectors for GAL4/C1-regulated expression are constructed. The three different vectors to test for each promoter are sense (+) expression, antisense (-) expression, and sense and antisense (+/-) RNA expression of a GL1 gene fragment. The (+) and (-) vectors are controls to compare for their effect on expression of GL1. In each case a 5' fragment from bases #739 to #1781 of the GL1 sequence (GenBank Accession M79448) are used for vector construction.

### GAL4/C1-regulated Expression

The GL1 gene fragments is cloned into the crossing-inducible vector construct pJG304-1 as *Ncol-Sacl* fragments. Plasmid pJG304 is derived from pBSSK+. Plasmid pBS SK+ (Stratagene, LaJolla, CA) is linearized with *Sacl,* treated with mung bean nuclease to remove the *Sacl* site, and re-ligated with T4 ligase to make pJG201. The 10XGAL4 consensus binding site/CaMV 35S minimal promoter/GUS gene/CaMV terminator cassette is removed from pAT71 with *Kpnl* and cloned into the *Kpnl* site of pJG201 to make pJG304. Plasmid pJG304 is partially digested with restriction endonuclease *Asp718* to isolate a full-length linear fragment. This fragment is ligated with a molar excess of the 22 base oligonucleotide JG-L (5'-GTA CCT CGA G TC TAG ACT CGA G-3', SEQ ID NO:5). Restriction analysis is used to identify a clone with this linker inserted 5' to the GAL4 DNA binding site, and this plasmid is designated pJG304DXhol.
The *Ncol* and *Sacl* sites are added to the ends of (+) and (-) fragments by synthesizing PCR primers with the appropriate restriction sites to the 5' termini. The (+/-) GL1 fragment is produced by first producing two fragment : a (+) fragment with the *Ncol* site at the 5' terminus and a *HindIII* site at the 3' terminus and a (-) fragment with a *HindIII* site at the 5' terminus and a *Sacl* site at the 3' terminus. The (+/-) unit is produced by ligation of the resulting fragments at the *EcoRI* site. The expression unit contains the GAL4 DNA binding domain, followed by a minimal TATA sequence, and the GL1 gene fragment oriented either (+), (-) or (+/-) (Guyer et al. (1998) Genetics, 149: 633-639).

### Constitutive Expression

The *mas 1'* promoter of mannopine synthase from Agrobacterium (Velten et al. (1984) EMBO Journal 3: 2723-2730), a relatively strong and constitutive in dicot plants is used. As above, the GL1 (+), (-), and (+/-) fragments are ligated behind the 1' promoter in pBluescript. The three different expression cassettes are ligated into pCIB200 as *EcoRI*/*SalI* fragments (Uknes et al. (1993) Plant Cell 5: 159-169).

### Example 3: Regulation of the Expression of the Cystathionine Beta-Lyase Gene

The Cystathionine Beta-Lyase (CBL) gene encodes a protein that carries out a step in the methionine biosynthesis pathway. CBL catalyzes the conversion of cystathionine to homocysteine (reviewed in Ravanel et al. (1998) *Proc. Natl. Acad, Sci, USA* 95: 7805-7812). The sequence of a cDNA for the *Arabidopsis* CBL gene has been identified (Ravanel et al. (1995) *Plant Mol. Biol.* 29: 875-882). The effect of the regulation of its expression in plants is tested using constructs for sense RNA expression (sense construct), antisense RNA expression (antisense construct) and sense and antisense RNA expression (sense/antisense construct).

### A. Antisense construct: binary BASTA vector pJG261 is used containing a fragment from the pJG304DXhol vector with an insertion of part of the CBL gene in an antisense orientation (nucleotides #13-1159, Genbank accession #L40511).

pJG304/aCBL: Plasmid pJG304DXhol is digested with *Nco*I and *Sac*l to excise the GUS gene. The GUS gene from pJG304DXhol is replaced with a CBL PCR product also digested with *Nco*I and *Sac*l. This product is generated using primers DG354 (5'-GAT CGA GCT CCA CGA GAA CTG TCT CCG-3'; SEQ ID NO:6) and DG357 (5'-TCA GCC ATG GGA AGA CAA GTA CAT TGC-3'; SEQ ID NO:7) and the pFL61 *Arabidopsis* cDNA library (Minet et al. (1992) *Plant J.* 2: 417-422) as a template. Plasmid pJG304/aCBL is constructed from the pJG304DXhol-digested vector ligated to the CBL PCR product.
pJG261/aCBL: pJG304/aCBL is cut with *Xhol* to excise the cassette containing the GAL4 DNA binding site/35S minimal promoter/antisense CBL/CaMV terminator fusion. This cassette is ligated into *Xho*l-digested pJG261 (Guyer, et al, *Genetics* (1998), 149: 633-639.), producing pJG261/aCBL.

### B. Sense construct: same as antisense construct, except the CBL fragment is in the opposite orientation. This construct contains the ATG start codon and most of the CBL ORF and serves as a control for regulation of the expression of the CBL gene.

pJG304/sCBL: Plasmid pJG304DXhol is digested with *Nco*I and Sad to excise the GUS gene. The GUS gene from pJG304DXhol is replaced with a CBL PCR product also digested with *Nco*l and *Sac*l. This product is generated using primers CBL1 (5'-CTT GCC ATG GCA CGA GAA CTG TCT CCG-3'; SEQ ID NO:8) and CBL2 (5'-CAT GGA GCT CGA AGA CAA GTA CAT TGC A-3'; SEQ ID NO:9) and the pFL61 *Arabidopsis* cDNA library as a template.

Plasmid pJG304/sCBL is constructed from the pJG304DXhol-digested vector ligated to the CBL PCR product.
pJG261/sCBL : pJG304/sCBL is cut with *Xho*l to excise the cassette containing the GAL4 DNA binding site/35S minimal promoter/sense CBL/CaMV terminator fusion. This cassette is ligated into *Xho*l-digested pJG261, producing pJG261/sCBL.

C. Sense/antisense construct: A CBL gene fragment (#13-1159) in the sense orientation is inserted into the *Sal*I site of vector pJG304DXhol downstream of the antisense orientation version of the CBL gene. A linker of about 10 bp is present between the two copies of CBL.
pJG304/dsCBL: Plasmid pJG304/aCBL is digested with *Sac*l. A CBL PCR product also digested with *Sac*l is inserted so that the inserted CBL gene is in the sense orientation. This product is generated using CBL2 (5'-CAT GGA GCT CGA AGA CAA GTA CAT TGC A-3'; SEQ ID NO:9) and CBL3 (5'-CAT CGA GCT CCT CTG TTT AAA CCA CGA GAA CTG TCT CCG TCG C-3'; SEQ ID NO:10) and the pFL61 *Arabidopsis* cDNA library as a template. The plasmid construct with the desired orientation of the inserted DNA is identified by digestion with *Hin*dIII. Plasmid pJG304/dsCBL is constructed from the pJG304/aCBL-digested vector ligated to the CBL PCR product. SURE2 (Stratagene, LaJolla, CA) is used as the bacterial host to stabilize the construct.
pJG261/dsCBL: pJG304/dsCBL is cut with Xbal to excise the cassette containing the GAL4 DNA binding site/353 minimal promoter/antisense CBUsense CBL/CaMV terminator fusion. This cassette is ligated into *Spe*l-digested pJG261, producing pJG261/dsCBL. XL1-BLUE MRF' (Stratagene, LaJolla, CA) is used as the bacterial host to partially stabilize the construct. Unrearranged DNA for this construct is isolated by agarose gel purification.

### D. Production of GAL4 Binding Site/Minimal CaMV 35S/CBL Transgenic Plants

The three described pJG261/CBL constructs are electro-transformed (Bio-Rad Laboratories, Hercules, CA) into *Agrobacterium tumefaciens recA*^{*-*} strain AGL1 (Lazo et al. (1991) *Bio*/*Technology* 9: 963-967), and *Arabidopsis* plants (Ecotype Columbia) are transformed by infiltration (Bechtold et al., (1993) *C. R. Acad. Sci. Paris,* 316: 1188-1193). Seeds from the infiltrated plants are selected on germination medium (Murashige-Skoog salts at 4.3 g/liter, Mes at 0.5 g/liter, 1% sucrose, thiamine at 10 ug/liter, pyridoxine at 5 ug/liter, nicotinic acid at 5 ug/liter, myo-inositol at 1 mg/liter, pH 5.8) containing Basta at 15 mg/liter.

### E. Comparison of the Inhibition of CBL Using a GAL4/C1 Transactivator and a GAL4 Binding Site/Minimal 35S Promoter

Transgenic plants containing a GAL4 binding site/minimal CaMV 35S promoter/ CBL construct are transplanted to soil and grown to maturity in the greenhouse. The presence of a transgenic CBL fragment in each line is confirmed by PCR. To test for the antisense construct, primers ASV1 (5'-TTT GGA GAG GAC AGA CCT GC-3'; SEQ ID NO:11) and CBL3 (5'-CAT CGA GCT CCT CTG TTT AAA CCA CGA GAA CTG TCT CCG TCG C-3'; SEQ ID NO:10) are used to verify the presence of an approximately 1200 bp product. Six transgenic lines with the antisense construct are identified. To test for the sense construct, primers ASV2 (5'-GGA TTT TGG TTT TAG GAA TTA GAA-3'; SEQ ID NO:12) and CBL3 (5'-CAT CGA GCT CCT CTG TTT AAA CCA CGA GAA CTG TCT CCG TCG C-3'; SEQ ID NO:10) are used to verify the presence of an approximately 1200 bp product. Thirteen transgenic lines with the sense construct are identified. To test for the sense/antisense construct, primers ASV2 (5'-GGA TTT TGG TTT TAG GAA TTA GAA-3'; SEQ ID NO:12) and CBL3 (5'-CAT CGA GCT CCT CTG TTT AAA CCA CGA GAA CTG TCT CCG TCG C-3'; SEQ ID NO: 10) are used to verify the presence of an approximately 1200 bp product. In addition, to test for the sense/antisense construct, primers ASV1 (5'-TTT GGA GAG GAC AGA CCT GC-3'; SEQ ID NO:11) and CBL3 (5'-CAT CGA GCT CCT CTG TTT AAA CCA CGA GAA CTG TCT CCG TCG C-3'; SEQ ID NO: 10) are used to verify the presence of an approximately 1200 bp product. Eleven transgenic lines with the sense/antisense construct are identified.
Flowers borne on the primary transformants are crossed to pollen from the homozygous GAL4/C1 transactivator line pAT53-103 (Guyer et al, *Genetics* (1998) 149: 633-649). F1 seeds are plated on MS + 2% sucrose medium (Murashige-Skoog salts at 4.3 g/liter, Mes at 0.5 g/liter, 2% sucrose). None of the lines comprising the antisense construct show an abnormal phenotype for the F1 progeny on plates. Two of thirteen lines comprising the sense construct show a weak phenotype for approximately half of the F1 progeny on each plate. The other eleven of thirteen lines comprising the sense construct do not show an abnormal phenotype for the F1 progeny on plates. Ten of eleven lines comprising the sense/antisense construct show phenotypes ranging from weak to strong for approximately half of the F1 progeny on each plate. Plants with a strong phenotype do not survive and have an increase in purple coloration, lose green pigmentation, and fail to form leaves after fourteen days on the plates. Plants with weaker phenotypes have some purple coloration, are paler green than normal, and form smaller leaves after fourteen days on the plates. Thus, a sense/antisense construct is more effective in decreasing the activity of an endogenous essential gene than either an antisense or a sense construct.

### F. Regulation of the Expression of CBL Using Two Promoters

The CBL gene is placed between two promoters - one producing transcription of the sense strand and the other producing transcription of the antisense strand. In this approach both sense and antisense RNA are produced in a plant cell and the two types of strands are capable of hybridizing to each other leading to the formation of double-stranded RNA molecules. The ACTIN2 is used as the promoter flanking both sides of the CBL gene. For all of these approaches, there is the option to use or not to use transcriptional terminators on the distal side of the promoters. If a transcriptional terminator is used, the transcript would start at the 3' end of one promoter, proceed through the CBL gene in one orientation, proceed through the second promoter from 3' end to 5' end, and terminate at the transcriptional terminator on the 5' end of the second promoter. Use of a transcriptional terminator may serve to stabilize the RNA transcribed.

### Example 4: Regulation of Luciferase Gene Expression

### Construction of a chimeric DNA molecule encoding sense and antisense luciferase RNA fragments (sense/antisense construct)

A 670 bp "sense" oriented fragment of the firefly luciferase gene from plasmid pluc+ (Promega vector pGL3, Genbank Accession # U47295) is amplified from pPH108 plasmid DNA using oligonucleotide primers ds_Luc1 (5'-CGC GGA TCC AAG ATT CAA AGT GCG CTG CTG-3', SEQ ID NO:13; *BamHI* restriction site underlined) and ds_Luc2 (5'-GCG AAG CTT GGC GAC GTA ATC CAC GAT CTC-3', SEQ ID NO:14; *HindIII* restriction site underlined). TurboPfu thermostable DNA polymerase (Stratagene) is used in 50 µl reactions according to the manufacturers protocol with five cycles of 95°C / 1 min, 55°C / 1.5 min, 72°C / 2 min followed by twenty five cycles of 95°C / 1 min, 72°C / 3.5 min. In a similar manner a 669 bp "antisense" oriented fragment of the firefly luciferase gene from plasmid pLuc+ is amplified by PCR from pPH108 plasmid DNA using oligonucleotide primers ds_Luc3 (5'-CGG TCT AGA AAG ATT CAA AGT GCG CTG CTG-3', SEQ ID NO:15; *Xbal* restriction site underlined) and ds_Luc2 (5'-GCG AAG CTT GGC GAC GTA ATC CAC GAT CTC-3', SEQ ID NO:16; *HindIII* restriction site underlined). The resulting DNA fragments are purified by electrophoresis through a 1% Tris-acetate gel made from low-melting point agarose (FMC) followed by phenol-chloroform extraction of the excised gel slices containing the PCR products. DNA from the sense product (ds_Luc1/2) is digested with *BamHI* and *Hindlll* and DNA from the antisense product (ds_Luc3/2) is digested with *Xbal* and *Hindlll* according to standard methods (restriction enzymes were obtained from New England Biolabs). The resulting sticky-ended DNA fragments are gel purified as described above.The sense *BamHI-HindIII* fragment and the antisense *HindIII-XbaI* fragment are then cloned in a three-way ligation into the cloning vector pLimus28 (New England Biolabs) which has been digested with *BamHI* and Xba/The resulting construct is named pAdF3. A 563 bp DNA fragment containing the open reading frame of the *bar* gene (D'Halluin et al. (1992) Methods in Enzymology 216:415-426) is amplified from plasmid CSA104 DNA using oligonucleotides bar-1 (5'-GCG **AAG CTT** GAT CCA TGA GCC CAG AAC GA-3', SEQ ID NO:17; *HindIII* restriction site bold) and bar-2 (5'-GCC **AAG CTT** CCT AGA ACG CGT GAT CTC-3', SEQ ID NO:18; *Hind III* restriction site bold). Pfu thermostable DNA polymerase (Stratagene) is used in 100 µl reaction according to the manufacturer's protocol with 25 cycles of 94°C / 1 min, 55°C/ 1 min, 72°C /1 min. Following a purification by phenol extraction and ethanol precipitation, the *bar* PCR product is digested with *Hindlll* and gel purified through a 2% agarose gel as described above. Plasmid pAdF1 is constructed by ligation of the *Hindlll* bar DNA fragment into plasmid pAdF3 which has been digested with *HindIII.* Of the two possible constructs resulting from this non-directional cloning, pAdF1 is characterized by the orientation of the *bar* gene, which is the same as that of the "sense" fragment of the luciferase gene present in pAdF3. A *BamHI-SaII* 1.2 kb DNA fragment containing the Arabidopsis ACT2 promoter (An et al., (1996) Plant J. 10:107-121) is excised from pNOV1416 (construct made by Scott Rabe; NB171 p.122; 8-17-98) and cloned into pUC21 digested with *BamHI* and *SaII.* to generate construct pAct2. The ACT2 promoter is then excised from pAct2 by digestion with *BamHI* and *Spel.*
In order to construct binary vector pAdF4 for Agrobacterium-mediated plant transformation with the ds_Luc1/2/3 RNA sense/antisense construct, DNA from binary plasmid pSGCHC1 carrying a kanamycin resistance gene for bacterial selection and a hygromycin resistance gene for transgenic plant selection is digested with. Spel and Sacl. The resulting 11.6 kb isolated fragment from pSGCHC1 is ligated in a three-way reaction using T4 DNA ligase (New England Biolabs) to the Act2 promoter *BamHI-Spel* fragment and the *BamHI-Sacl* fragment from pAdF3 which contains both sense and antisense luciferase gene fragments.

Similarly, the binary vector pAdF2 was constructed in a three-way ligation including the 11.6 kb pSGCHC1 vector digested with *Spel* and *Sacl* described above, the *Spel-BamHI* fragment carrying the Act2 promoter and the *BamHI-Sacl* fragment from pAdF1 which contains the sense and antisense luciferase gene fragments surrounding the bar gene.

### Transformation of Agrobacterium and Vacuum-infiltration of Arabidopsis plants

Plasmids pAdF2 and pAdF4 are introduced into *Agrobacterium tumafaciens* GV3101 by electroporation and transformed colonies selected and amplified. Four to five week old plants of *Arabidopsis thaliana* mutant lines expressing luciferase either constitutively (UBQ3 promoter (Norris et al. (1993) PMB 21: 895-906) / UBQ3 + CaMV 35S 5' UTR/luc+; *pPH108)* or inducibly (Arabidopsis PR-1 promoter/luc+;*pClB*200, *line* 6E) are vacuum infiltrated with Agrobacterium clones carrying the pAdF2 or pAdF4 binary T-DNA vectorsTransformed plants are co-selected on hygromycin and kanamycin and grown under controlled phytotron conditions for determination of luciferase activity. In addition, luciferase activity in the *pCIB200*-6E background is assessed 48 hr after induction with BTH (BTH treatment essentially as described in Lawton et al. (1996) Plant J. 10: 71-82). Luciferase activity is quantified using a luminescence-based assay in tissue extracts following the addition of luciferin substrate. Luciferase activity is also monitored *in planta* using a CCD-cooled video imaging system (Hamamatsu). Note that the luciferase gene in pPH108 is from pGL3 (Promega, Genbank Accession # U47295); and the luciferase gene in pCIB200-6E is from pGL2 (Promega, Genbank Accession # X65323).
Luciferase assays are conducted with a luciferase assay reagent (Promega, catalog number E1501). Leaf punches are ground in 0.1M KPO₄ pH7.8 buffer at 4°C and spun down briefly. An aliquot of the supernatant is mixed with the luciferase assay reagent, and luciferase activity is quantified in a Monolight 2010 luminometer (Becton Dickinson Microbiology System). Results for the analysis of pPH108 plants transformed with sense/antisense constructs pAdF2 and pAdF4 are presented in Table 1, as well as results for pCIB200-6E plants transformed with sense/antisense construct pAdF4 are shown in Table 2.

**Table 1:**

| Relative Light Units for luciferase activity in plants. | | | | | |
|---|---|---|---|---|---|
| Different plants of parent line pPH108 are tested to determine the average level of luciferase in various plants. This line is not homozygotic, therefore individual plants showing the highest levels of luciferase (i.e. plants 1,2 and 3) may be homozygotic. Independent T1 lines of double transgenic plants pPH108(pAdF2), which comprise the pAdF2 construct in a pPH108 background, or pPH108(pAdF4), which comprise the pAdF4 construct in a pPH108 background,, are analyzed. Luciferase levels vary from line to line probably due to a range of expression levels of the sense/antisense construct. Independent T1 events, wild-type Arabidopsis Columbia plants transformed with either pAdF2 (Columbia(pAdF2)) or pAdF4 (Columbia(pAdF4)), are also analyzed as controls. | | | | | |

| **Plant#** | **pPH108** | **pPH108 (pAdF2)** | **pPH108 (pAdF4)** | **Columbia (pAdF2)** | **Columbia (pAdF4)** |
|---|---|---|---|---|---|
| 1 | 449102 | 108452 | 338274 | 390 | 425 |
| 2 | 381191 | 85301 | 116609 | 282 | 339 |
| 3 | 338480 | 21013 | 90545 | 231 | 273 |
| 4 | 199141 | 9669 | 81241 | 160 | 242 |
| 5 | 190187 | 8098 | 54457 | 150 | 226 |
| 6 | 137125 | 7349 | 32361 | 144 | 214 |
| 7 | 134421 | 6197 | 23929 | 133 | 213 |
| 8 | 124596 | 4944 | 18780 | 133 | 192 |
| 9 | 121062 | 4482 | 16637 | 129 | 179 |
| 10 | 121047 | 3975 | 15008 | 127 | 161 |
| 11 | 115430 | 3697 | 14901 | 127 | 161 |
| 12 | 114916 | 3244 | 13342 | 123 | 156 |
| 13 | 106007 | 3146 | 13111 | 122 | 156 |
| 14 | 104298 | 2516 | 12198 | 121 | 151 |
| 15 | 92455 | 2449 | 9538 | 118 | 138 |
| 16 | | 2315 | 8316 | 107 | 131 |
| 17 | | 1850 | 8301 | | |
| 18 | | 1689 | 5957 | | |
| 19 | | 1578 | 5110 | | |
| 20 | | 1282 | 4941 | | |
| 21 | | 859 | 4904 | | |
| 22 | | 202 | 4754 | | |
| 23 | | 155 | 4642 | | |
| 24 | | | 4147 | | |
| 25 | | | 3296 | | |
| 26 | | | 3286 | | |
| 27 | | | 2885 | | |
| 28 | | | 2800 | | |
| 29 | | | 2423 | | |
| 30 | | | 1703 | | |

**Table 2:**

| Relative Light Units for the luciferase activity in individual plants tested. | | | |
|---|---|---|---|
| Plantlets from the parent line pCIB200-6E are grown and sampled as well as independent T1 double transgenic lines pCIB200-6E(pAdF4). Independent T1 events, wild-type Arabidopsis Columbia transformed with construct pAdF4, are also analyzed as controls. | | | |

| **Plant#** | **pCIB200-6E** | **pCIB200-6E(pAdF4)** | **Columbia(pAdF4)** |
|---|---|---|---|
| 1 | 17983 | 19990 | 214 |
| 2 | 7662 | 5157 | 217 |
| 3 | 6657 | 4753 | 233 |
| 4 | 5608 | 3334 | 263 |
| 5 | 3340 | 1370 | 186 |
| 6 | 2850 | 736 | 291 |
| 7 | 2461 | 484 | 239 |
| 8 | 2445 | 478 | 227 |
| 9 | 2349 | 476 | 274 |
| 10 | 2301 | 373 | |

### Example 5: Regulation of the Expression of a Gene of Interest Using a Site-Specific Recombination System

In another embodiment of the invention, sense and antisense RNA fragments are produced using a site-specific recombination system. Site-specific recombination is a process of DNA cleavage and religation mediated by an enzyme recombinase. Recombinase recognizes a highly specific DNA sequence. Some site-specific recombination systems use only one protein to act on its target site. Several site-specific recombination systems such as Cre/lox, FLP/FRT, R/RS and Gin/gix are known to mediate site-specific recombination processes in plants (Ow and Medberry, 1995, Critical Reviews in Plant Sciences 14:239-261). Dependent on the configuration of recombinase recognition sites in the recombination substrates the recombination process results in deletion, inversion or integration of DNA sequences. One particular reaction of this kind, i.e. inversion, is related to this invention. Two recombinase recognition sites are placed in the desirable gene expression cassette in opposite orientations. The first recognition site is placed downstream of the promoter either in the untranslated leader or inside the 5'-proximal region of the transgene coding sequence. The second site is placed in the 3'-untranslated region or in the 3'-proximal region of the transgene coding sequence in the opposite orientation to the first site. The coding or noncoding strand of the desired DNA sequence is operably linked to a promoter. In the presence of the recombinase, the sequences flanked by the two recognition sites in opposite orientation are inverted. As a consequence of the recombination process, both coding and non-coding strands of the desired genes are operably linked to the promoter and both sense and antisense transcripts are produced from the same genetic loci in the same chromosomal environment and are capable of forming dsRNA molecules.
High level constitutive expression of site-specific recombinase such as Cre can cause abnormal phenotypes in the some plants (Que et al., 1998, Plant J. 13:401-409). Preferably expression of the recombinase is under the control of an inducible promoter. In one embodiment of the invention, the recombinase expression cassette is placed in the same transformation vector used for introducing the desirable transgene sequences flanked by two recombinase recognition sites in opposite orientation. In another embodiment of the invention, the transgenic plants containing the transgene flanked by the recombinase recognition sites are crossed to lines expressing recombinase or re-transformed with a recombinase-expressing vector. In another embodiment of the invention, a recombinant RNA or DNA virus containing the inducible recombinase expression cassette is used to infect plants containing the desirable transgene construct. The recombinant virus is preferably defective in causing abnormal phenotypes. In the above embodiments the recombinase causes the inversion of the chromosomal copy of the desirable sequences, thereby producing the desired dsRNA.

In yet another embodiment of the invention, a recombinant plant DNA virus is used to produce sense and antisense RNA fragments capable of forming a dsRNA. Typically plant DNA viruses replicate epi-chromosomally. In a preferred embodiment, a construct containing a cassette comprising a DNA sequence capable of expressing a RNA fragment of a target gene flanked by two inverted recombinase recognition sites is introduced into plant cells along with a second recombinant virus expressing a recombinase. Alternatively, both the recombinase expression cassette and the DNA sequence flanked by inverted recognition sites are linked in the same viral DNA vector. After the viral DNA vectors are introduced into plants, the recombinase gene is expressed. Expression of the recombinase gene causes inversion of the viral DNA sequences flanked by recombinase recognition sites and results in the production of large amount of dsRNA from the desired DNA sequences due to the high copy numbers of viral genome in the cell. Preferred recombinase systems are Cre/lox, FLP/FRT, R/RS and Gin/gix are known to mediate site-specific recombination processes in plants (Ow and Medberry, 1995, Critical Reviews in Plant Sciences 14:239-261, incorporated herein by reference).

### Example 6: Requirements for Construction of Plant Expression Cassettes

Gene sequences intended for expression in transgenic plants are firstly assembled in expression cassettes behind a suitable promoter and upstream of a suitable transcription terminator. All requirement for constructions of plant expression cassettes apply to the DNA molecules of the present invention and are carried out using techniques well-known in the art.

### Promoter Selection

The selection of promoter used in expression cassettes determines the spatial and temporal expression pattern of the DNA molecule in the transgenic plant. Selected promoters express DNA molecule in specific cell types (such as leaf epidermal cells, mesophyll cells, root cortex cells) or in specific tissues or organs (roots, leaves or flowers, for example) and this selection reflects the desired location of biosynthesis of a RNA fragment encoded by the DNA molecule. Alternatively, the selected promoter may drive expression of the DNA molecule under a light-induced or other temporally regulated promoter. A further alternative is that the selected promoter be chemically regulated. This provides the possibility of inducing the expression of the DNA molecule only when desired and caused by treatment with a chemical inducer.

### Transcriptional Terminators

A variety of transcriptional terminators are available for use in expression cassettes. These are responsible for the termination of transcription and, preferably, correct polyadenylation.

Appropriate transcriptional terminators and those which are known to function in plants and include the CaMV 35S terminator, the *tml* terminator, the nopaline synthase terminator, the pea *rbcS* E9 terminator. These can be used in both monocotyledons and dicotyledons.

### Sequences for the Enhancement or Regulation of Expression

Numerous sequences have been found to enhance gene expression from within the transcriptional unit and these sequences can be used in conjunction with the DNA molecule of this invention to increase its expression in transgenic plants.
Various intron sequences have been shown to enhance expression, particularly in monocotyledonous cells. For example, the introns of the maize *Adh1* gene have been found to significantly enhance the expression of the wild-type gene under its cognate promoter when introduced into maize cells. Intron 1 is found to be particularly effective and enhanced expression in fusion constructs with the chloramphenicol acetyltransferase gene (Callis *et al*., Genes Develep 1: 1183-1200 (1987)). In the same experimental system, the intron from the maize *bronze1* gene had a similar effect in enhancing expression (Callis *et al., supra).* Intron sequences have been routinely incorporated into plant transformation vectors, typically within the non-translated leader.
A number of non-translated leader sequences derived from viruses are also known to enhance expression, and these are particularly effective in dicotyledonous cells. Specifically, leader sequences from Tobacco Mosaic Virus (TMV, the "Ω-sequence"), Maize Chlorotic Mottle Virus (MCMV), and Alfalfa Mosaic Virus (AMV) have been shown to be effective in enhancing expression (*e*.*g*. Gallie *et al.* Nucl. Acids Res. 15: 8693-8711 (1987); Skuzeski *et al.* Plant Molec. Biol. 15; 65-79 (1990)).

### Example 7: Examples of Expression Cassette Construction

The present invention encompasses the expression of a DNA molecule of the present invention under the regulation of any promoter which is expressible in plants, regardless of the origin of the promoter. Therefore the DNA molecule is inserted into any of the expression cassette using techniques well-known in the art. These expression cassettes can then be easily transferred to the plant transformation vectors described below.
Furthermore, the invention also encompasses the use of any plant-expressible promoter in conjunction with any further sequences required or selected for the expression of the DNA molecule. Such sequences include, but are not restricted to, transcriptional terminators, extraneous sequences to enhance expression (such as introns *[e.g. Adh* intron 1], viral sequences [*e. g.* TMV-Ω]).

### Constitutive Expression: the CaMV 35S Promoter

Construction of the plasmid pCGN1761 is described in the published patent application EP 0 392 225. pCGN1761 contains the "double" 35S promoter and the *tml* transcriptional terminator with a unique *EcoRl* site between the promoter and the terminator and has a pUC-type backbone. A derivative of pCGN1761 is constructed which has a modified polylinker which includes *Notl* and *Xhol* sites in addition to the existing *EcoRI* site. This derivative is designated pCGN1761ENX. pCGN1761ENX is useful for the cloning of cDNA sequences or gene sequences (including microbial ORF sequences) within its polylinker for the purposes of their expression under the control of the 35S promoter in transgenic plants. The entire 35S promoter-gene sequence-tml terminator cassette of such a construction can be excised by *HindIII, Sphl, SaII,* and *Xbal* sites 5' to the promoter and *Xbal, BamHI* and *BgII* sites 3' to the terminator for transfer to transformation vectors. Furthermore, the double 35S promoter fragment can be removed by 5' excision with *HindIII, Sphl, SalI, Xbal,* or *Pstl,* and 3' excision with any of the polylinker restriction sites *(EcoRI, Notl* or *Xhol)* for replacement with another promoter.
Accordingly, a DNA molecule of the present invention is inserted into pCGN1761ENX for constitutive expression under the control of the CaMV 35S promoter.

### Expression under a Chemically Regulatable Promoter

This section describes the replacement of the double 35S promoter in pCGN1761ENX with any promoter of choice; by way of example the chemically regulated PR-1a promoter is described. The promoter of choice is preferably excised from its source by restriction enzymes, but can alternatively be PCR-amplified using primers which carry appropriate terminal restriction sites. Should PCR-amplification be undertaken, then the promoter should be resequenced to check for amplification errors after the cloning of the amplified promoter in the target vector. The chemically regulatable tobacco PR-1a promoter is cleaved from plasmid pCIB1004 (see EP 0 332 104) and transferred to plasmid pCGN1761ENX. pCIB1004 is cleaved with *Ncol* and the resultant 3' overhang of the linearized fragment is rendered blunt by treatment with T4 DNA polymerase. The fragment is then cleaved with *HindIII* and the resultant PR-1a promoter containing fragment is gel purified and cloned into pCGN1761 ENX from which the double 35S promoter has been removed. This is done by cleavage with *XhoI* and blunting with T4 polymerase, followed by cleavage with *Hindlll* and isolation of the larger vector-terminator containing fragment into which the pCIB1004 promoter fragment is cloned. This generates a pCGN1761ENX derivative with the PR-1a promoter and the *tml* terminator and an intervening polylinker with unique *EcoRI* and *Notl* sites.
A DNA molecule of the present invention is inserted into this vector, and the fusion product (*i.e.* promoter-gene-terminator) is subsequently transferred to any selected transformation vector, including those described in this application, thus providing for chemically inducible expression of the DNA molecule.

### Constitutive Expression: the Actin Promoter

Several isoforms of actin are known to be expressed in most cell types and consequently the actin promoter is a good choice for a constitutive promoter. In particular, the promoter from the rice *Act1* gene has been cloned and characterized (McElroy *et al.* Plant Cell 2: 163-171 (1990)). A 1.3 kb fragment of the promoter is found to contain all the regulatory elements required for expression in rice protoplasts. Furthermore, numerous expression vectors based on the *Act1* promoter have been constructed specifically for use in monocotyledons (McElroy *et al.* Mol. Gen. Genet. 231: 150-160 (1991)). These incorporate the *Act1*-intron 1, *Adh1* 5' flanking sequence and *Adh1*-intron 1 (from the maize alcohol dehydrogenase gene) and sequence from the CaMV 35S promoter. Vectors showing highest expression are fusions of 35S and the *Act1* intron or the *Act1* 5' flanking sequence and the *Act1* intron. The promoter expression cassettes described by McElroy et *al.* (Mol. Gen. Genet. 231: 150-160 (1991)) is easily modified for the expression of a DNA molecule of the present invention and are particularly suitable for use in monocotyledonous hosts. For example, promoter containing fragments are removed from the McElroy constructions and used to replace the double 35S promoter in pCGN1761ENX, which is then available for the insertion or specific gene sequences. The fusion genes thus constructed are transferred to appropriate transformation vectors. In a separate report the rice *Act1* promoter with its first intron has also been found to direct high expression in cultured barley cells (Chibbar *et al.* Plant Cell Rep. 12: 506-509 (1993)).
A DNA molecule of the present invention is inserted downstream of such promoter, and the fusion products *(i.e,* promoter-gene-terminator) are subsequently transferred to any selected transformation vector, including those described in this application.

### Constitutive Expression: the Ubiquitin Promoter

Ubiquitin is another gene product known to accumulate in many cell types and its promoter has been cloned from several species for use in transgenic plants (*e.g.* sunflower - Binet *et al.* Plant Science 79: 87-94 (1991), maize - Christensen *et al*. Plant Molec. Biol. 12: 619-632 (1989)). The maize ubiquitin promoter has been developed in transgenic monocot systems and its sequence and vectors constructed for monocot transformation are disclosed in the patent publication EP 0 342 926. Further, Taylor *et al.* (Plant Cell Rep. 12: 491-495 (1993)) describe a vector (pAHC25) which comprises the maize ubiquitin promoter and first intron and its high activity in cell suspensions of numerous monocotyledons when introduced via microprojectile bombardment. The ubiquitin promoter is clearly suitable for the expression of a DNA molecule of the present invention in transgenic plants, especially monocotyledons. Suitable vectors are derivatives of pAHC25 or any of the transformation vectors described in this application, modified by the introduction of the appropriate ubiquitin promoter and/or intron sequences.
A DNA molecule of the present invention is therefore inserted into any of these vector, and the fusion products (*i.e.* promoter-gene-terminator) are used for transformation of plants, resulting in constitutive expression of the DNA molecule.

### Root Specific Expression

A preferred pattern of expression for a DNA molecule of the instant invention is root expression. Expression of the nucleotide sequence only in root tissue has the advantage of altering the expression of a target gene only in roots, without a concomitant alteration of its expression in leaf and flower tissue and seeds. A suitable root promoter is that described by de Framond (FEBS 290: 103-106 (1991)) and also in the published patent application EP 0 452 269. This promoter is transferred to a suitable vector such as pCGN1761ENX and the DNA molecule is inserted into such vector. The entire promoter-gene-terminator cassette is subsequently transferred to a transformation vector of interest.

### Wound Inducible Promoters

Numerous such promoters have been described (*e.g.* Xu *et al.* Plant Molec. Biol. 22: 573-588 (1993), Logemann *et al.* Plant Cell 1: 151-158 (1989), Rohrmeier & Lehle, Plant Molec. Biol. 22: 783-792 (1993), Firek *et al.* Plant Molec. Biol. 22: 129-142 (1993), Warner *et al.* Plant J. 3: 191-201 (1993)) and all are suitable for use with the instant invention. Logemann *et al. (supra)* describe the 5' upstream sequences of the dicotyledonous potato *wun1* gene. Xu *et al. (supra)* show that a wound inducible promoter from the dicotyledon potato *(pin2)* is active in the monocotyledon rice. Further, Rohrmeier & Lehle *(supra)* describe the cloning of the maize *Wip1* cDNA which is wound induced and which can be used to isolated the cognate promoter using standard techniques. Similarly, Firek *et al. (supra)* and Warner *et al. (supra)* have described a wound induced gene from the monocotyledon *Asparagus officinalis* which is expressed at local wound and pathogen invasion sites. Using cloning techniques well known in the art, these promoters can be transferred to suitable vectors, fused to a DNA molecule of this invention, and used to express these genes at the sites of insect pest infection.

### Pith Preferred Expression

Patent application WO 93/07278 describes the isolation of the maize *trpA* gene which is preferentially expressed in pith cells. Using standard molecular biological techniques, this promoter or parts thereof, can be transferred to a vector such as pCGN1761 where it can replace the 35S promoter and be used to drive the expression of a DNA molecule of the present invention in a pith-preferred manner. In fact, fragments containing the pith-preferred promoter or parts thereof are transferred to any vector and modified for utility in transgenic plants. Pith preferred expression of the DNA molecule is achieved by inserting the DNA molecule in such vector.

### Pollen-Specific Expression

Patent Application WO 93/07278 further describes the isolation of the maize calcium-dependent protein kinase (CDPK) gene which is expressed in pollen cells. The gene sequence and promoter extend up to 1400 bp from the start of transcription. Using standard molecular biological techniques, this promoter or parts thereof, is transferred to a vector such as pCGN1761 where it replaces the 35S promoter and is used to drive the expression of a DNA molecule of the present invention in a pollen-specific manner. In fact fragments containing the pollen-specific promoter or parts thereof can be transferred to any vector and modified for utility in transgenic plants.

### Leaf-Specific Expression

A maize gene encoding phosphoenol carboxylase (PEPC) has been described by Hudspeth & Grula (Plant Molec Biol 12: 579-589 (1989)). Using standard molecular biological techniques the promoter for this gene is used to drive the expression of a DNA molecule of the present invention in a leaf-specific manner in transgenic plants.

### Example 8: Construction of Plant Transformation Vectors

Numerous transformation vectors are available for plant transformation, and a DNA molecule of this invention is inserted into any of the expression cassettes described above, such that they are capable of expressing the DNA molecule in desirable cells, under appropriate conditions. A nucleotide sequence-containing expression cassette is then incorporated into any appropriate transformation vector described below.
The selection of vector for use will depend upon the preferred transformation technique and the target species for transformation. For certain target species, different antibiotic or herbicide selection markers may be preferred. Selection markers used routinely in transformation include the *nptII* gene which confers resistance to kanamycin and related antibiotics (Messing & Vierra, Gene 19: 259-268 (1982); Bevan *et al.,* Nature 304:184-187 (1983)), the *bar* gene which confers resistance to the herbicide phosphinothricin (White *et al.,* Nucl Acids Res 18: 1062 (1990), Spencer *et al.* Theor Appl Genet 79: 625-631 (1990)), the *hph* gene which confers resistance to the antibiotic hygromycin (Blochinger & Diggelmann, Mol Cell Biol 4: 2929-2931), and the *dhfr* gene, which confers resistance to methotrexate (Bourouis *et al.,* EMBO J. 2(7): 1099-1104 (1983)).

### (1) Construction of Vectors Suitable for Agrobacterium Transformation

Many vectors are available for transformation using *Agrobacterium tumefaciens.* These typically carry at least one T-DNA border sequence and include vectors such as pBIN19 (Bevan, Nucl. Acids Res. (1984). Below the construction of two typical vectors is described.

### Construction of pCIB200 and pCIB2001

The binary vectors pCIB200 and pCIB2001 are used for the construction of recombinant vectors for use with *Agrobacterium* and is constructed in the following manner. pTJS75kan is created by *Narl* digestion of pTJS75 (Schmidhauser & Helinski, J Bacteriol. 164: 446-455 (1985)) allowing excision of the tetracycline-resistance gene, followed by insertion of an *Accl* fragment from pUC4K carrying an NPTII (Messing & Vierra, Gene 19: 259-268 (1982); Bevan *et al.*, Nature 304: 184-187 (1983); McBride *et al.,* Plant Molecular Biology 14: 266-276 (1990)). *Xhol* linkers are ligated to the *EcoRV* fragment of pCIB7 which contains the left and right T-DNA borders, a plant selectable *nos*/*nptII* chimeric gene and the pUC polylinker (Rothstein *et al.,* Gene 53: 153-161 (1987)), and the *Xhol*-digested fragment is cloned into *Sall*-digested pTJS75kan to create pCIB200 (see also EP 0 332 104). pCIB200 contains the following unique polylinker restriction sites: *EcoRI, Sstl, Kpnl, BgIII, Xbal,* and *Sall. pCIB2001* is a derivative of pCIB200 which created by the insertion into the polylinker of additional restriction sites. Unique restriction sites in the polylinker of pCIB2001 are *EcoRI, Sstl, Kpnl, Bglll, Xbal, Sall, Mlul, Bcll, AvrII, Apal, Hpal,* and *Stul.* pCIB2001, in addition to containing these unique restriction sites also has plant and bacterial kanamycin selection, left and right T-DNA borders for *Agrobacterium*-mediated transformation, the RK2-derived *trfA* function for mobilization between *E. coli* and other hosts, and the *OriT* and *OriV* functions also from RK2. The pCIB2001 polylinker is suitable for the cloning of plant expression cassettes containing their own regulatory signals.
Any one of the plant expression cassettes described above and comprising a DNA molecule of the present invention are inserted into pCIB2001, preferably using the polylinker.

### Construction of pCIB10 and Hygromycin Selection Derivatives thereof

The binary vector pCIB10 contains a gene encoding kanamycin resistance for selection in plants, T-DNA right and left border sequences and incorporates sequences from the wide host-range plasmid pRK252 allowing it to replicate in both *E. coli* and *Agrobacterium.* Its construction is described by Rothstein *et al.* (Gene 53: 153-161 (1987)). Various derivatives of pCIB10 have been constructed which incorporate the gene for hygromycin B phosphotransferase described by Gritz *et al.* (Gene 25: 179-188 (1983)). These derivatives enable selection of transgenic plant cells on hygromycin only (pCIB743), or hygromycin and kanamycin (pCIB715, pCIB717). This vectors is used transform an expression cassette comprising a DNA molecule of the present invention.

### (2) Construction of Vectors Suitable for non-Agrobacterium Transformation.

Transformation without the use of *Agrobacterium tumefaciens* circumvents the requirement for T-DNA sequences in the chosen transformation vector and consequently vectors lacking these sequences can be utilized in addition to vectors such as the ones described above which contain T-DNA sequences. Transformation techniques which do not rely on *Agrobacterium* include transformation via particle bombardment, protoplast uptake *(e.g.* PEG and electroporation), microinjection or pollen transformation (US Patent 5,629,183).

The choice of vector depends largely on the preferred selection for the species being transformed. Below, the construction of some typical vectors is described.

### Construction of pCIB3064

pCIB3064 is a pUC-derived vector suitable for direct gene transfer techniques in combination with selection by the herbicide basta (or phosphinothricin). The plasmid pCIB246 comprises the CaMV 35S promoter in operational fusion to the *E. coli* GUS gene and the CaMV 35S transcriptional terminator and is described in the PCT published application WO 93/07278. The 35S promoter of this vector contains two ATG sequences 5' of the start site. These sites are mutated using standard PCR techniques in such a way as to remove the ATGs and generate the restriction sites *Sspl* and *Pvull*. The new restriction sites are 96 and 37 bp away from the unique *Sall* site and 101 and 42 bp away from the actual start site. The resultant derivative of pCIB246 is designated pCIB3025. The GUS gene is then excised from pCIB3025 by digestion with *Sall* and *Sacl,* the termini rendered blunt and religated to generate plasmid pCIB3060. The plasmid pJIT82 is obtained from the John Innes Centre, Norwich and the a 400 bp *Smal* fragment containing the *bar* gene from *Streptomyces viridochromogenes* is excised and inserted into the *Hpal* site of pCIB3060 (Thompson *et al.* EMBO J 6: 2519-2523 (1987)). This generated pCIB3064 which comprises the *bar* gene under the control of the CaMV 35S promoter and terminator for herbicide selection, a gene for ampicillin resistance (for selection in *E. coli)* and a polylinker with the unique sites *Sphl, Pstl, Hindlll,* and *BamHl.* This vector is suitable for the cloning of plant expression cassettes containing their own regulatory signals to direct expression of a DNA molecule of the present invention.

### Construction of pSOG19 and pSOG35

pSOG35 is a transformation vector which utilizes the *E. coli* gene dihydrofolate reductase (DHFR) as a selectable marker conferring resistance to methotrexate. PCR is used to amplify the 35S promoter (∼800 bp), intron 6 from the maize Adh1 gene (∼550 bp) and 18 bp of the GUS untranslated leader sequence from pSOG10. A 250 bp fragment encoding the *E. coli* dihydrofolate reductase type II gene is also amplified by PCR and these two PCR fragments are assembled with a *Sacl-Pstl* fragment from pBI221 (Clontech) which comprised the pUC19 vector backbone and the nopaline synthase terminator. Assembly of these fragments generated pSOG19 which contains the 35S promoter in fusion with the intron 6 sequence, the GUS leader, the DHFR gene and the nopaline synthase terminator. Replacement of the GUS leader in pSOG19 with the leader sequence from Maize Chlorotic Mottle Virus (MCMV) generated the vector pSOG35. pSOG19 and pSOG35 carry the pUC gene for ampicillin resistance and have *HindIII, Sphl, PstI* and *EcoRl* sites available for the cloning of foreign sequences, in particular a DNA molecule of the present invention.

### Example 9: Chloroplast Transformation

### Transformation vectors

For expression of a DNA molecule of the present invention in plant plastids, plastid transformation vector pPH143 (WO 97/32011, example 36) is used. The DNA molecule is inserted into pPH143 thereby replacing the PROTOX coding sequence. This vector is then used for plastid transformation and selection of transformants for spectinomycin resistance. Alternatively, the DNA molecule is inserted in pPH143 so that it replaces the aadH gene. In this case, transformants are selected for resistance to PROTOX inhibitors.

### Chloroplast Transformation

Seeds of *Nicotiana tabacum* c.v. 'Xanthi nc' were germinated seven per plate in a 1" circular array on T agar medium and bombarded 12-14 days after sowing with 1 *µ*m tungsten particles (M10, Biorad, Hercules, CA) coated with DNA from plasmids pPH143 and pPH145 essentially as described (Svab, Z. and Maliga, P. (1993) *PNAS* 90, 913-917). Bombarded seedlings were incubated on T medium for two days after which leaves were excised and placed abaxial side up in bright light (350-500 µmol photons/m²/s) on plates of RMOP medium (Svab, Z., Hajdukiewicz, P. and Maliga, P. (1990) *PNAS* 87, 8526-8530) containing 500 µg/ml spectinomycin dihydrochloride (Sigma, St. Louis, MO). Resistant shoots appearing underneath the bleached leaves three to eight weeks after bombardment were subcloned onto the same selective medium, allowed to form callus, and secondary shoots isolated and subcloned. Complete segregation of transformed plastid genome copies (homoplasmicity) in independent subclones was assessed by standard techniques of Southern blotting (Sambrook et al., (1989) Molecular Cloning: A Laboratory Manual, Cold Spring Harbor Laboratory, Cold Spring Harbor). BamHI/EcoRI-digested total-cellular DNA (Mettler, I. J. (1987) *Plant Mol Biol Reporter* 5, 346-349) was separated on 1% Tris-borate (TBE) agarose gels, transferred to nylon membranes (Amersham) and probed with ³²P-labeled random primed DNA sequences corresponding to a 0.7 kb BamHI/HindIII DNA fragment from pC8 containing a portion of the *rps7*/*12* plastid targeting sequence. Homoplasmic shoots are rooted aseptically on spectinomycin-containing MS/IBA medium (McBride, K. E. et al. (1994) *PNAS* 91, 7301-7305) and transferred to the greenhouse.

## Claims

1. A method for altering expression of a target gene in a plant cell, comprising introducing into a plant cell a sense RNA fragment of said target gene and an antisense RNA fragment of said target gene, wherein said sense RNA fragment and said antisense RNA fragment are capable of forming a double-stranded RNA molecule.

2. The method of claim 1, wherein said RNA fragments are comprised in two different RNA molecules.

3. The method of claim 1, wherein said RNA fragments are comprised in one RNA molecule.

4. The method of claim 3, wherein said RNA molecule is capable of folding such that said RNA fragments comprised therein form a double-stranded region.

5. A method for altering expression of a target gene in a plant cell, comprising introducing into a plant cell a first DNA sequence capable of expressing in said cell a sense RNA fragment of said target gene and a second DNA sequence capable of expressing in said cell an antisense RNA fragment of said target gene, wherein (i) said first DNA sequence and said second DNA sequence are comprised in two different DNA molecules; and (ii) said first DNA sequence and said second DNA sequence are stably integrated in the genome of said plant cell; and (iii) said sense RNA fragment and said antisense RNA fragment are capable of forming a double-stranded RNA molecule.

6. The method of claim 5, wherein said target gene is an essential gene of said plant cell.

7. The method of claim 5, wherein said target gene is a heterologous gene stably integrated in the genome of said plant cell.

8. The method of claim 7, wherein said heterologous gene is present in said plant cell as an extrachromosomal molecule.

9. The method of claim 5, wherein said DNA molecules further comprise a first promoter operably linked to said first DNA sequence and a second promoter operably linked to said second DNA sequence.

10. A plant cell comprising a first DNA sequence capable of expressing a sense RNA fragment of a target gene and a second DNA sequence capable of expressing an antisense RNA fragment of said target gene, wherein (i) said first DNA sequence and said second DNA sequence are comprised in two different DNA molecules; and (ii) said first DNA sequence and said second DNA sequence are stably integrated in the genome of said plant cell; and (iii) said sense RNA fragment and said antisense RNA fragment are capable of forming a double-stranded RNA molecule and expression of said target gene in said plant cell is altered, when said DNA sequences are expressed.

11. A plant and the progeny thereof derived from the plant cell of claim 10, wherein said plant and the progeny thereof comprise a first DNA sequence capable of expressing a sense RNA fragment of a target gene and a second DNA sequence capable of expressing an antisense RNA fragment of said target gene, wherein (i) said first DNA sequence and said second DNA sequence are comprised in two different DNA molecules; and (ii) said first DNA sequence and said second DNA sequence are stably integrated in the genome of said plant and the progeny thereof; and (iii) said sense RNA fragment and said antisense RNA fragment are capable of forming a double-stranded RNA molecule and expression of said target gene in said plant and the progeny thereof is altered, when said DNA sequences are expressed.

12. Seeds derived from the plant of claim 11 wherein said seeds comprise a first DNA sequence capable of expressing a sense RNA fragment of a target gene and a second DNA sequence capable of expressing an antisense RNA fragment of said target gene, wherein (i) said first DNA sequence and said second DNA sequence are comprised in two different DNA molecules; and (ii) said first DNA sequence and said second DNA sequence are stably integrated in the genome of said seed; and (iii) said sense RNA fragment and said antisense RNA fragment are capable of forming a double-stranded RNA molecule and expression of said target gene in said seed is altered, when said DNA sequences are expressed.

## Patentansprüche

1. Verfahren zur Veränderung der Expression eines Zielgens in einer Pflanzenzelle, umfassend das Einführen, in eine Pflanzenzelle, eines sense-RNA-Fragments des Zielgens und eines antisense-RNA-Fragments des Zielgens, worin das sense-RNA-Fragment und das antisense-RNA-Fragment ein doppelsträngiges RNA-Molekül bilden können.

2. Verfahren gemäss Anspruch 1, worin die RNA-Fragmente in zwei unterschiedlichen RNA-Molekülen enthalten sind.

3. Verfahren gemäss Anspruch 1, worin die RNA-Fragmente in einem RNA-Molekül enthalten sind.

4. Verfahren gemäss Anspruch 3, worin das RNA-Molekül sich so falten kann, dass die darin enthaltenen RNA-Fragmente eine doppelsträngige Region bilden.

5. Verfahren zur Veränderung der Expression eines Zielgens in einer Pflanzenzelle, umfassend das Einführen, in eine Pflanzenzelle, einer ersten DNA-Sequenz, die ein sense-RNA-Fragment des Zielgens in der Zelle exprimieren kann, und einer zweiten DNA-Sequenz, die ein antisense-RNA-Fragment des Zielgens in der Zelle exprimieren kann, worin (i) die erste DNA-Sequenz und die zweite DNA-Sequenz in zwei unterschiedlichen DNA-Molekülen enthalten sind; und (ii) die erste DNA-Sequenz und die zweite DNA-Sequenz stabil im Genom der Pflanzenzelle integriert sind; und (iii) das sense-RNA-Fragment und das antisense-RNA-Fragment ein doppelsträngiges RNA-Molekül bilden können.

6. Verfahren gemäss Anspruch 5, worin das Zielgen ein essentielles Gen der Pflanzenzelle ist.

7. Verfahren gemäss Anspruch 5, worin das Zielgen ein heterologes Gen ist, das stabil im Genom der Pflanzenzelle integriert ist.

8. Verfahren gemäss Anspruch 7, worin das heterologe Gen in der Pflanzenzelle als extrachromosomales Molekül vorhanden ist.

9. Verfahren gemäss Anspruch 5, worin die DNA-Moleküle ferner einen ersten Promotor, der funktionsfähig mit der ersten DNA-Sequenz verbunden ist, und einen zweiten Promotor umfassen, der funktionsfähig mit der zweiten DNA-Sequenz verbunden ist.

10. Pflanzenzelle, die eine erste DNA-Sequenz, die ein sense-RNA-Fragment eines Zielgens exprimieren kann, und eine zweite DNA-Sequenz umfasst, die ein antisense-RNA-Fragment des Zielgens exprimieren kann, worin (i) die erste DNA-Sequenz und die zweite DNA-Sequenz in zwei unterschiedlichen DNA-Molekülen enthalten sind; und (ii) die erste DNA-Sequenz und die zweite DNA-Sequenz stabil im Genom der Pflanzenzelle integriert sind; und (iii) das sense-RNA-Fragment und das antisense-RNA-Fragment ein doppelsträngiges RNA-Molekül bilden können und die Expression des Zielgens in der Pflanzenzelle verändert ist, wenn die DNA-Sequenzen exprimiert werden.

11. Pflanze und Nachkommenschaft davon, die aus der Pflanzenzelle gemäss Anspruch 10 stammt, worin die Pflanze und die Nachkommenschaft davon eine erste DNA-Sequenz, die ein sense-RNA-Fragment eines Zielgens exprimieren kann, und eine zweite DNA-Sequenz umfassen, die ein antisense-RNA-Fragment des Zielgens exprimieren kann, worin (i) die erste DNA-Sequenz und die zweite DNA-Sequenz in zwei unterschiedlichen DNA-Molekülen enthalten sind; und (ii) die erste DNA-Sequenz und die zweite DNA-Sequenz stabil im Genom der Pflanze und der Nachkommenschaft davon integriert sind; und (iii) das sense-RNA-Fragment und das antisense-RNA-Fragment ein doppelsträngiges RNA-Molekül bilden können und die Expression des Zielgens in der Pflanze und in der Nachkommenschaft davon verändert ist, wenn die DNA-Sequenzen exprimiert werden.

12. Samen, die aus der Pflanze gemäss Anspruch 11 stammen, worin die Samen eine erste DNA-Sequenz, die ein sense-RNA-Fragment eines Zielgens exprimieren kann, und eine zweite DNA-Sequenz umfassen, die ein antisense-RNA-Fragment des Zielgens exprimieren kann, worin (i) die erste DNA-Sequenz und die zweite DNA-Sequenz in zwei unterschiedlichen DNA-Molekülen enthalten sind; und (ii) die erste DNA-Sequenz und die zweite DNA-Sequenz stabil im Genom des Samens integriert sind; und (iii) das sense-RNA-Fragment und das antisense-RNA-Fragment ein doppelsträngiges RNA-Molekül bilden können und die Expression des Zielgens im Samen verändert ist, wenn die DNA-Sequenzen exprimiert werden.

## Revendications

1. Procédé d'altération de l'expression d'un gène cible dans une cellule végétale, comprenant l'introduction dans une cellule végétale d'un fragment d'ARN sens dudit gène cible et d'un fragment d'ARN antisens dudit gène cible, ledit fragment d'ARN sens et ledit fragment d'ARN antisens étant capables de former une molécule d'ARN double brin.

2. Procédé selon la revendication 1, dans lequel lesdits fragments d'ARN sont contenus dans deux molécules d'ARN différentes.

3. Procédé selon la revendication 1, dans lequel lesdits fragments d'ARN sont contenus dans une seule molécule d'ARN.

4. Procédé selon la revendication 3, dans lequel ladite molécule d'ARN est capable de se replier de façon que lesdits fragments d'ARN qui y sont contenus forment une région double brin.

5. Procédé d'altération de l'expression d'un gène cible dans une cellule végétale, comprenant l'introduction dans une cellule végétale d'une première séquence d'ADN capable d'exprimer dans ladite cellule un fragment d'ARN sens dudit gène cible et une deuxième séquence d'ADN capable d'exprimer dans ladite cellule un fragment d'ARN antisens dudit gène cible, où (i) ladite première séquence d'ADN et ladite deuxième séquence d'ADN sont contenues dans deux molécules d'ADN différentes; et (ii) ladite première séquence d'ADN et ladite deuxième séquence d'ADN sont intégrées de manière stable dans le génome de ladite cellule végétale; et (iii) ledit fragment d'ARN sens et ledit fragment d'ARN antisens sont capables de former une molécule d'ARN double brin.

6. Procédé selon la revendication 5, dans lequel ledit gène cible est un gène essentiel de ladite cellule végétale.

7. Procédé selon la revendication 5, dans lequel ledit gène cible est un gène hétérologue intégré de manière stable dans le génome de ladite cellule végétale.

8. Procédé selon la revendication 7, dans lequel ledit gène hétérologue est présent dans ladite cellule végétale sous forme d'une molécule extrachromosomique.

9. Procédé selon la revendication 5, dans lequel lesdites molécules d'ADN comprennent en outre un premier promoteur fonctionnellement lié à ladite première séquence d'ADN et un deuxième promoteur fonctionnellement lié à ladite deuxième séquence d'ADN.

10. Cellule végétale comprenant une première séquence d'ADN capable d'exprimer un fragment d'ARN sens d'un gène cible et une deuxième séquence d'ADN capable d'exprimer un fragment d'ARN antisens dudit gène cible, où (i) ladite première séquence d'ADN et ladite deuxième séquence d'ADN sont contenues dans deux molécules d'ADN différentes, et (ii) ladite première séquence d'ADN et ladite deuxième séquence d'ADN sont intégrées de manière stable dans le génome de ladite cellule végétale; et (iii) ledit fragment d'ARN sens et ledit fragment d'ARN antisens sont capables de former une molécule d'ARN double brin, et l'expression dudit gène cible dans ladite cellule végétale est altérée lorsque lesdites séquences d'ADN sont exprimées.

11. Plante et sa descendance, issues de la cellule végétale de la revendication 10, ladite plante et sa descendance comprenant une première séquence d'ADN capable d'exprimer un fragment d'ARN sens d'un gène cible et une deuxième séquence d'ADN capable d'exprimer un fragment d'ARN antisens dudit gène cible, où (i) ladite première séquence d'ADN et ladite deuxième séquence d'ADN sont contenues dans deux molécules d'ADN différentes; et (ii) ladite première séquence d'ADN et ladite deuxième séquence d'ADN sont intégrées de manière stable dans le génome de ladite plante et de sa descendance; et (iii) ledit fragment d'ARN sens et ledit fragment d'ARN antisens sont capables de former une molécule d'ARN double brin, et l'expression dudit gène cible dans ladite plante et dans sa descendance est altérée lorsque lesdites séquences d'ADN sont exprimées.

12. Graines provenant de la plante de la revendication 11, lesdites graines comprenant une première séquence d'ADN capable d'exprimer un fragment d'ARN sens d'un gène cible et une deuxième séquence d'ADN capable d'exprimer un fragment d'ARN antisens dudit gène cible, où (i) ladite première séquence d'ADN et ladite deuxième séquence d'ADN sont contenues dans deux molécules d'ADN différentes; et (ii) ladite première séquence d'ADN et ladite deuxième séquence d'ADN sont intégrées de manière stable dans le génome de ladite graine; et (iii) ledit fragment d'ARN sens et ledit fragment d'ARN antisens sont capables de former une molécule d'ARN double brin, et l'expression dudit gène cible dans ladite graine est altérée lorsque lesdites séquences d'ADN sont exprimées.
